# EUROPÄISCHE PATENTSCHRIFT

(11) **EP 3 104 900 B1**
(45) Veröffentlichungstag und Bekanntmachung des Hinweises auf die Patenterteilung: **01.04.2020**
(21) Anmeldenummer: 15704006.4
(22) Anmeldetag: 12.02.2015
(51) Int. Cl.: A61L 15/22, A61L 15/42, A61L 15/64

(54) **MEDIZINISCHES PRODUKT UND VERFAHREN ZU SEINER HERSTELLUNG**
MEDICAL PRODUCT AND METHOD FOR THE PRODUCTION THEREOF
PRODUIT MEDICAL ET PROCEDE DE FABRICATION CORRESPONDANT

(30) Priorität: 12.02.2014 DE 102014202578
(43) Veröffentlichungstag der Anmeldung: 21.12.2016
(73) Patentinhaber: Aesculap AG, 78532 Tuttlingen (DE); Carl Freudenberg KG, 69469 Weinheim (DE)
(72) Erfinder: ODERMATT, Erich, CH-8200 Schaffhausen (CH); BARGON, Rainer, 78532 Tuttlingen (DE); GRAFAHREND, Dirk, 68165 Mannheim (DE); NEUMÜLLER, Daniel, 69469 Weinheim (DE); REIBEL, Denis, F-67850 Herrlisheim (FR)
(74) Vertreter: Patentanwälte Ruff, Wilhelm, Beier, Dauster & Partner mbB
(86) Internationale Anmeldenummer: PCT/EP2015/052966
(87) Internationale Veröffentlichungsnummer: WO 2015/121350

(56) Entgegenhaltungen:
- EP-A2- 2 042 199
- WO-A1-2010/132636
- WO-A1-2012/055494
- DE-A1-102010 012 845
- US-A1- 2009 136 651
- JOHN L ET AL: "In vitro hydrolytic degradation of centrifugally spun polyhydroxybutyrate?pectin composite fibres", POLYMER INTERNATIONAL, BARKING, GB, Bd. 58, 1. Januar 2009 (2009-01-01), Seiten 1442-1451, XP007910705, DOI: 10.1002/PI.2682 [gefunden am 2009-09-29]
- MOHAMMAD REZA BADROSSAMAY ET AL: "Nanofiber Assembly by Rotary Jet-Spinning", NANO LETTERS, Bd. 10, Nr. 6, 9. Juni 2010 (2010-06-09), Seiten 2257-2261, XP055057406, ISSN: 1530-6984, DOI: 10.1021/nl101355x
- L. Amalorpava Mary ET AL: "Centrifugal spun ultrafine fibrous web as a potential drug delivery vehicle", eXPRESS Polymer Letters, vol. 7, no. 3, 1 January 2013 (2013-01-01) , pages 238-248, XP055464663, ISSN: 1788-618X, DOI: 10.3144/expresspolymlett.2013.22
- BADROSSAMAY MOHAMMAD R ET AL: "Engineering hybrid polymer-protein super-aligned nanofibers via rotary jet spinning", BIOMATERIALS, ELSEVIER SCIENCE PUBLISHERS BV., BARKING, GB, vol. 35, no. 10, 20 January 2014 (2014-01-20), pages 3188-3197, XP028822053, ISSN: 0142-9612, DOI: 10.1016/J.BIOMATERIALS.2013.12.072
- Hadi Hajiali ET AL: "Electrospun PGA/gelatin nanofibrous scaffolds and their potential application in vascular tissue engineering", INTERNATIONAL JOURNAL OF NANOMEDICINE, 1 January 2011 (2011-01-01), page 2133, XP055464669, AUCKLAND, NZ ISSN: 1176-9114, DOI: 10.2147/IJN.S24312

## Beschreibung

Die Erfindung betrifft ein medizinisches Produkt in Form einer vliesförmigen Wundauflage, umfassend rotationsgesponnene Fasern, sowie ein Herstellungsverfahren für das medizinische Produkt.

Medizinische Produkte in Form von Vliesstoffen oder Faser-Wirrgelegen mit rotationsgesponnenen Gelatinefasern sowie deren Verwendung zur Wundheilung oder Wundabdeckung sind aus den Druckschriften WO 2008/107126 A1 und WO 2009/036958 A2 bekannt. Eine Vorrichtung zur Erzeugung solcher rotationsgesponnener Fasern ist in der DE 10 2005 048 939 A1 beschrieben.

Aus der WO 2012/022422 A1 sind Vliesstoffe mit Hydroxypropylcellulosefasern unter anderem zur Wundversorgung oder zur Verwendung als Wundauflage bekannt.

Die Verwendung von modifizierter Stärke zur Blutstillung ist aus der WO 2009/091549 A1 bekannt.

Körperhygieneartikel mit schmelzgesponnenen Kompositstärkefasern gehen aus der US 2012/ 0216709 A1 hervor.

Mehrlagige Duraersatzmaterialien auf Basis von Kollagen sind aus den Druckschriften US 5,997,895 A, WO 2007/082295 A2 und EP 2 147 687 A2 bekannt. Weiterhin ist in der EP 1 025 870 A1 ein Duraersatzprodukt auf Basis eines Copolymers aus Caprolacton und Lactid beschrieben.

Aus der DE 196 54 884 A1 ist eine Abdeckmembran für die Geweberegeneration bekannt, die aus einem Kunststoffmaterial sowie einem natürlichen Material, insbesondere Kollagen, besteht.

Ferner sind rotationsgesponnene, vliesförmige Wundauflagen bekannt (Badrossamay MR et al.: Nanofiber Assembly by Rotary Jet-Spinning. Nano Lett. 2010; 10, 2257-2261 und EP 2 042 199 A2).

Aus der WO 2012/055494 A1 ist ein Vliesstoff bekannt, dessen Fasern unterschiedliche polymere Bestandteile aufweisen.

Ferner sind tubuläre Gerüststrukturen, welche elektrogesponnene Fasern aus Polyglykolsäure und Gelatine aufweisen, bekannt (Hajiali, Hadi et al. "Electrospun PGA/gelatin nanofibrous scaffolds and their potential application in vascular tissue engineering." International Journal of Nanomedicine vol. 6 (2011): 2133-2141).

Für die Gewebeversiegelung kommen grundsätzlich die folgenden Produktkategorien zum Einsatz:
Die erste Kategorie betrifft Versiegelungsmittel auf Basis von Polysacchariden, die innerhalb kurzer Zeit eine große Menge Flüssigkeit aufnehmen und die Blutgerinnung durch Konzentration von Blutplättchen beschleunigen können. Derartige Versiegelungsmittel sind beispielsweise unter den Bezeichnungen Tabotamp®, Surgicel®, ChitoFlex® oder Perclot® kommerziell erhältlich.

Die zweite Kategorie von Versiegelungsmitteln betrifft Kollagenprodukte, die ähnlich wirken wie die Versiegelungsmittel der ersten Kategorie, allerdings zusätzlich in der Diskussion stehen, die Gerinnungskaskade durch Aktivierung des Faktors Xlla zu stimulieren. Entsprechende Produkte werden beispielsweise unter den Bezeichnungen Spongostan® (Schweinegelatine), Lyostypt® und DuraGen® (jeweils bovines Kollagen) kommerziell vertrieben.

Die dritte Kategorie betrifft flüssige Versiegelungsmittel, die meistens auf synthetischen Polymeren basieren. Beispiele hierfür sind Hydrogele auf Basis von Polyethylenglykol-Derivaten, die zum Beispiel unter den Bezeichnungen Coseal®, Progel®, Duraseal®, Focalseal® oder Bioglue® kommerziell erhältlich sind.

Die letzte Kategorie bilden Fibrinkleber. Im Unterschied zu den zuvor beschriebenen Versiegelungsmitteln greifen Fibrinkleber aktiv in die Gerinnungskaskade ein und basieren auf einem Zusammenspiel der Komponenten Fibrin, Thrombin und Aprotinin, wodurch eine Blutkoagulation bewirkt wird. Entsprechende Produkte sind beispielsweise unter den Bezeichnungen Evicel®, Tissucol®, Quixil® oder Beriplast® kommerziell erhältlich.

Obgleich sich mit gattungsgemäßen Gewebeversiegelungsmitteln grundsätzlich zufriedenstellende Ergebnisse erzielen lassen, kann es vereinzelt zu Problemen kommen.

Bei Versiegelungsmitteln auf Basis von oxidierter Cellulose können beispielsweise das Auftreten von Infektionen und Gewebereizungen sowie die Bildung saurer Abbauprodukte problematisch und nachteilig für den Heilungsverlauf sein. Ein weiterer Nachteil ist der in der Regel sehr aufwändige und zudem umweltschädliche Herstellungsprozess, bei welchem Cellulosevliese mit giftigem, karzinogenem Stickstoffdioxid behandelt werden. Bereits geringste Abweichungen können dabei zu einer Überoxidation und damit zu einem vollständigen Verlust der mechanischen Stabilität führen.

Versiegelungsmittel auf Basis von Chitosan haben den grundsätzlichen Nachteil, dass Chitosan aus den Panzern von Meereskrustentieren gewonnen werden muss, was den Herstellungsprozess kostspielig macht. Ferner ist Chitosan in vivo nur begrenzt abbaubar.

Pulverförmige Hämostyptika können insofern nachteilig sein, als sie bei besonders starken Blutungen aufgrund des Fehlens einer geschlossenen Hämostyptikaschicht von einem Wundsitus weggespült werden können. Zudem ist eine Kompression von Blutgefäßen durch den alleinigen Einsatz von Pulvern in der Regel nicht möglich.

Bei Kollagenprodukten sind in der Regel aufwändige Aufreinigungsverfahren und Verfahren zum Nachweis einer vollständigen Virus- und Prionendeaktivierung erforderlich. Ferner besteht ein gewisses Risiko von proteinbasierten Allergien. Zudem können Kollagenimplantate bisweilen ursächlich für Kalzifizierungen sein.

Flüssige Gewebeversiegelungsmittel müssen meist in aufwändigen Synthesen hergestellt werden, was deren Produktion verteuert. Zudem müssen diese Versiegelungsmittel in der Regel kühl gelagert und über Mehrkammersysteme ausgetragen werden. Für einen Chirurgen bedeutet dies zusätzliche Arbeitsschritte und einen höheren Zeitaufwand. Viele flüssige Versiegelungsmittel weisen außerdem nach der Aushärtung eine geringe Reißfestigkeit auf.

Fibrinkleber sind in der Regel teuer. Zu deren Gewinnung kommen üblicherweise speziell validierte Methoden auf Basis von Humanblut zum Einsatz, insbesondre um das Risiko einer Ansteckung mit HIV, Hepatitis C oder Creutzfeldt-Jakob zu minimieren. Fibrinkleber werden ebenfalls über Mehrkammersysteme ausgetragen und weisen daher insoweit die gleichen, im vorangegangenen Absatz beschriebenen Nachteile auf. Zudem ist die Haltbarkeit von Fibrinklebern stark eingeschränkt.

### Aufgabe und Lösung

Aufgabe der vorliegenden Erfindung ist es daher, ein medizinisches Produkt bereitzustellen, welches aus dem Stand der Technik bekannte Probleme umgeht und insbesondere zur Versiegelung von Wunden eingesetzt werden kann.

Diese Aufgabe wird gelöst durch ein medizinisches Produkt mit den Merkmalen des unabhängigen Anspruchs 1 und ein Verfahren zur Herstellung eines solchen Produkts mit den Merkmalen des Anspruchs 13. Bevorzugte Ausführungsformen des medizinischen Produktes sind in den abhängigen Ansprüchen 2 bis 12 definiert. Der Wortlaut sämtlicher Ansprüche wird hiermit durch ausdrückliche Bezugnahme zum Inhalt der vorliegenden Beschreibung gemacht.

Gemäß einem ersten Erfindungsaspekt schlägt die Erfindung ein medizinisches Produkt vor, welches rotationsgesponnene, d.h. mittels Rotation gesponnene, Fasern umfasst. Das medizinische Produkt kann insbesondere als Vliesstoff ausgebildet sein.

Die Fasern enthalten wenigstens zwei unterschiedliche Polymere, nämlich wenigstens ein synthetisches und bioresorbierbares Polymer, welches vorzugsweise zusätzlich hydrophob ist, sowie wenigstens ein weiteres Polymer, welches hydrophil und/oder gewebeadhäsiv ist.

Überraschenderweise hat sich herausgestellt, dass sich synthetische und bioresorbierbare Polymere zusammen mit hydrophilen und/oder gewebeadhäsiven Polymeren mittels Rotation zu multifunktionalen, insbesondere bifunktionalen, Medizinprodukten ausspinnen lassen, welche grundsätzlich ein breites medizinisches Anwendungsgebiet eröffnen, aufgrund der erfindungsgemäß vorgesehenen Polymere jedoch insbesondere für die Wundversiegelung geeignet sind.

So erlaubt die Verwendung von hydrophilen Polymeren die Ausbildung von flüssigkeitsaufnehmenden Eigenschaften, was insbesondere im Hinblick auf eine rasche Blutstillung von Vorteil ist. Die Verwendung von Polymeren, welche zusätzlich oder alternativ hierzu gewebeadhäsive Eigenschaften besitzen, begünstigt die Ausbildung von dislokationsbeständigen, abdichtenden, mechanisch stabilisierenden sowie insbesondere als Keimbarriere wirkenden Produkten, was wiederum insbesondere vorteilhaft im Hinblick auf einen dichten und dauerhaften Wundverschluss sowie auf die Vermeidung post-chirurgischer Infektionen ist.

Durch die Verwendung von synthetischen und gleichzeitig bioresorbierbaren Polymeren ist zudem sichergestellt, dass erfindungsgemäße Produkte wenigstens teilweise in vivo resorbierbar sind.

Die oben beschriebenen Polymere erlauben zudem mit besonderem Vorteil weitere Verwendungen des medizinischen Produktes, worauf im Folgenden noch näher eingegangen werden wird.

Das Rotationsspinnen hat zudem den Vorteil, dass es eine Fülle weiterer Parameter bereithält, um die Beschaffenheit der Fasern und damit die Eigenschaften des medizinischen Produktes gezielt zu beeinflussen. Beispielsweise lassen sich durch die Festlegung der Rotationsgeschwindigkeit, eines Material- und/oder Luftgradienten, der Austragslochgröße im Boden eines Rotationsbehältnisses (Spinnrotors) sowie durch das Anlegen eines elektrischen Feldes eine weitaus größere Bandbreite an Faserdurchmessern verwirklichen als dies in der Regel bei konventionellen Schmelzspinnverfahren der Fall ist. Aufgrund der großen Zahl variierbarer Parameter können erfindungsgemäße medizinische Produkte mit besonderem Vorteil für spezielle Anwendungen maßgeschneidert werden. Hierdurch lassen sich beispielsweise medizinische Produkte mit hoher Reißfestigkeit (Höchstzugspannung) und insbesondere hoher Elastizität herstellen.

Ein weiterer Vorteil besteht darin, dass das Rotationsspinnverfahren relativ einfach zu kontrollieren ist. Die physikalischen Gesetzmäßigkeiten der für die Faserbildung verantwortlichen Zentripetalkräfte sorgen stets für reproduzierbare Herstellungsbedingungen. Diese Herstellungsbedingungen lassen sich auch bei der Realisierung größerer Produktionsmaßstäbe (sogenanntes Upscaling), beispielsweise durch den Einsatz größerer Rotationsbehältnisse, ohne weiteres anpassen.

Schließlich liegt ein besonderer Vorteil darin, dass sich erfindungsgemäße Produkte, insbesondere aufgrund der in den vorangegangenen Abschnitten erwähnten Eigenschaften, durch hervorragende Handhabungseigenschaften auszeichnen.

Der Ausdruck "synthetisch" meint im Sinne der vorliegenden Erfindung, dass das entsprechende Polymer nicht natürlich hergestellt ist, sondern seine Herstellung in der Regel auf einer chemischen, insbesondere industriellen oder technischen, oder rekombinanten, d.h. mittels rekombinanter Organismen bewirkten, Synthese beruht.

Der Ausdruck "bioresorbierbar" bedeutet im Sinne der vorliegenden Erfindung, dass das entsprechende Polymer in vivo abgebaut und resorbiert wird. Der Abbau vollzieht sich dabei vorzugsweise ohne die Entstehung von immunogenen oder toxischen Abbauprodukten.

Der Ausdruck "hydrophil" bedeutet im Sinne der vorliegenden Erfindung, dass das entsprechende Polymer allgemein wasserliebend ist, d.h. eine starke Wechselwirkung mit Wasser, insbesondere über Wasserstoffbrückenbindungen, eingeht, und entspricht damit dem üblichen fachmännischen Verständnis. Insbesondere definiert der Ausdruck "hydrophil" im Sinne der vorliegenden Erfindung, dass das entsprechende Polymer in Wasser löslich oder quellbar ist. Entsprechend bedeutet der Ausdruck "hydrophob" im Sinne der vorliegenden Erfindung, dass das entsprechende Polymer wasserabweisend ist, und steht somit ebenfalls im Einklang mit der üblichen fachmännischen Bedeutung.

Der Ausdruck "gewebeadhäsiv" bedeutet im Sinne der vorliegenden Erfindung, dass das entsprechende Polymer in der Lage ist, mit biologischen, insbesondere menschlichen oder tierischen, Gewebeschichten, beispielsweise einer Mucinschicht von Schleimhäuten, eine adhäsive Bindung einzugehen. Die adhäsive Bindung beruht vorzugsweise ausschließlich auf Van-der-Waals-Wechselwirkungen, elektrostatischen Wechselwirkungen und/oder Dipol-Dipol-Wechselwirkungen. Mit anderen Worten zeichnet sich ein gewebeadhäsives Polymer im Sinne der vorliegenden Erfindung vorzugsweise dadurch aus, dass es befähigt ist, eine adhäsive Bindung mit Geweben einzugehen, die nicht auf der Ausbildung kovalenter Bindungen beruht.

In einer bevorzugten Ausführungsform umfasst das medizinische Produkt rotationsgesponnene Fasern, die einen im Verhältnis zueinander unterschiedlichen Anteil an dem wenigstens einen synthetischen und bioresorbierbaren Polymer und/oder dem wenigstens einen hydrophilen und/oder gewebeadhäsiven Polymer enthalten. Dadurch können die vorstehend beschriebenen multifunktionalen Eigenschaften des Produktes stärker zur Geltung gebracht werden.

Das medizinische Produkt weist in einer weiteren Ausführungsform rotationsgesponnene Fasern auf, welche einen höheren Anteil an dem wenigstens einen synthetischen und bioresorbierbaren Polymer aufweisen als an dem wenigstens einen hydrophilen und/oder gewebeadhäsivem Polymer.

In einer ergänzenden oder alternativen Ausführungsform weist das medizinische Produkt rotationsgesponnene Fasern auf, welche einen kleineren Anteil an dem wenigstens einen synthetischen und bioresorbierbaren Polymer aufweisen als an dem wenigstens einen hydrophilen und/oder gewebeadhäsiven Polymer.

In einer weiteren Ausführungsform umfasst das medizinische Produkt ferner rotationsgesponnene Fasern, welche wenigstens ein synthetisches und bioresorbierbares Polymer im Sinne der vorliegenden Erfindung, jedoch kein hydrophiles und/oder gewebeadhäsives Polymer im Sinne der vorliegenden Erfindung enthalten. Die Fasern der in diesem Absatz beschriebenen Ausführungsform bilden bevorzugt eine Außenflächenschicht des erfindungsgemäßen Produktes.

In einer weiteren Ausführungsform umfasst das medizinische Produkt ferner rotationsgesponnene Fasern, welche wenigstens ein hydrophiles und/oder gewebeadhäsives Polymer im Sinne der vorliegenden Erfindung, jedoch kein synthetisches und bioresorbierbares Polymer im Sinne der vorliegenden Erfindung enthalten. Die Fasern der in diesem Absatz beschriebenen Ausführungsform bilden bevorzugt ebenfalls eine Außenflächenschicht des erfindungsgemäßen Produktes, insbesondere eine Außenflächenschicht, welche der in dem vorangegangenen Abschnitt erwähnten Außenflächenschicht gegenüberliegt.

Das medizinische Produkt weist rotationsgesponnene Faserschichten, vorzugsweise rotationsgesponnene Faserfraktionen auf, die sich in Bezug auf den Faseranteil des wenigstens einen synthetischen und bioresorbierbaren Polymers und/oder des wenigstens einen hydrophilen und/oder gewebeadhäsiven Polymers voneinander unterscheiden. Aufgrund der schichtförmigen Ausbildung solcher Fasern lassen sich die multifunktionalen Eigenschaften des Produktes noch stärker zur Geltung bringen. Insbesondere ist hierdurch ein medizinisches Produkt mit funktional unterscheidbaren Faserfraktionen, insbesondere Faserschichten, realisierbar.

Das medizinische Produkt weist in einer weiteren Ausführungsform wenigstens eine rotationsgesponnene Faserschicht auf, deren Fasern einen höheren Anteil an dem wenigstens einen synthetischen und bioresorbierbaren Polymer enthalten als an dem wenigstens einen hydrophilen und/oder gewebeadhäsiven Polymer. Bei der wenigstens einen Faserschicht handelt es sich vorzugsweise um eine Außenflächenschicht des Produktes.

In einer ergänzenden oder alternativen Ausführungsform umfasst das medizinische Produkt wenigstens eine rotationsgesponnene Faserschicht, deren Fasern einen kleineren Anteil an dem wenigstens einen synthetischen und bioresorbierbaren Polymer enthalten als an dem wenigstens einen hydrophilen und/oder gewebeadhäsiven Polymer. Bei der wenigstens einen Faserschicht handelt es sich vorzugsweise um eine Außenflächenschicht des Produktes, insbesondere um eine Außenflächenschicht, welche der im vorangegangenen Abschnitt erwähnten Außenflächenschicht gegenüberliegt.

Erfindungsgemäß kann es weiterhin vorgesehen sein, dass das medizinische Produkt ferner wenigstens eine rotationsgesponnene Faserschicht umfasst, deren Fasern das wenigstens eine synthetische und bioresorbierbare Polymer, jedoch nicht das wenigstens eine hydrophile und/oder gewebeadhäsive Polymer enthalten. Mit anderen Worten kann es erfindungsgemäß vorgesehen sein, dass das medizinische Produkt ferner wenigstens eine rotationsgesponnene Faserschicht umfasst, deren Fasern wenigstens ein synthetisches und bioresorbierbares Polymer im Sinne der vorliegenden Erfindung, jedoch kein hydrophiles und/oder gewebeadhäsives Polymer im Sinne der vorliegenden Erfindung enthalten. Bei der wenigstens einen Faserschicht handelt es sich vorzugsweise ebenfalls um eine Außenflächenschicht des Produktes, insbesondere um eine Außenflächenschicht, welche einer der in den vorangegangenen Abschnitten erwähnten Außenflächenschichten gegenüberliegt.

In einer weiteren Ausführungsform umfasst das Produkt ferner wenigstens eine rotationsgesponnene Faserschicht, deren Fasern das wenigstens eine hydrophile und/oder gewebeadhäsive Polymer, jedoch nicht das wenigstens eine synthetische und bioresorbierbare Polymer enthalten. Mit anderen Worten umfasst das Produkt in einer weiteren Ausführungsform ferner wenigstens eine rotationsgesponnene Faserschicht, deren Fasern wenigstens ein hydrophiles und/oder gewebeadhäsives Polymer im Sinne der vorliegenden Erfindung, jedoch kein synthetisches und bioresorbierbares Polymer im Sinne der vorliegenden Erfindung enthalten. Bei der wenigstens einen Faserschicht handelt es sich vorzugsweise ebenso um eine Außenflächenschicht des Produktes, insbesondere um eine Außenflächenschicht, welche einer der in den vorangegangenen Abschnitten erwähnten Außenflächenschichten, vorzugsweise der im vorangegangenen Abschnitt erwähnten Außenflächenschicht, gegenüberliegt.

Gemäß einer besonders bevorzugten Ausführungsform weist das medizinisches Produkt einen Faseranteilsgradient in Bezug auf das wenigstens eine synthetische und bioresorbierbare Polymer und/oder das wenigstens eine hydrophile und/oder gewebeadhäsive Polymer, besonders bevorzugt in Bezug auf das wenigstens eine synthetische und bioresorbierbare Polymer und das wenigstens eine hydrophile und/oder gewebeadhäsive Polymer, auf. Dadurch ist mit besonderem Vorteil eine vorzugsweise graduelle Einstellung funktionaler Produkteigenschaften, wie beispielsweise der Bioresorbierbarkeit, der Reißfestigkeit, der Flüssigkeitsaufnahmefähigkeit, des Gewebeadhäsionsvermögen und dergleichen, möglich.

Der Anteil des wenigstens einen synthetischen und bioresorbierbaren Polymers und/oder des wenigstens einen hydrophilen und/oder gewebeadhäsiven Polymers in den Fasern kann sich in einer weiteren Ausführungsform von einer ersten Außenfläche des Produktes in Richtung einer zweiten, vorzugsweise gegenüberliegenden Außenfläche des Produktes, ändern, vorzugsweise graduell ändern, d.h. einem Gradienten folgen. Mit anderen Worten ist ein Faseranteilsgradient vorzugsweise entlang der Dicke des Produktes ausgebildet. Auf diese Weise lassen sich funktionale Produkteigenschaften, wie beispielsweise Bioresorbierbarkeit, Reißfestigkeit, Flüssigkeitsaufnahmefähigkeit, Gewebeadhäsionsvermögen und dergleichen, entlang der Dicke des Produktes vorzugsweise graduell einstellen.

Bevorzugt nimmt der Anteil des wenigstens einen synthetischen und bioresorbierbaren Polymers in den Fasern von der ersten Außenfläche des Produktes in Richtung der zweiten, vorzugsweise gegenüberliegenden Außenfläche des Produktes zu, vorzugsweise graduell zu, und/oder der Anteil des wenigstens einen hydrophilen und/oder gewebeadhäsiven Polymers in den Fasern von der ersten Außenfläche des Produktes in Richtung der zweiten, vorzugsweise gegenüberliegenden Außenfläche des Produktes ab, vorzugsweise graduell ab.

Besonders bevorzugt nimmt der Anteil des wenigstens einen synthetischen und bioresorbierbaren Polymers in den Fasern von der ersten Außenfläche des Produktes in Richtung der zweiten, vorzugsweise gegenüberliegenden Außenfläche des Produktes zu, vorzugsweise graduell zu, und entsprechend der Anteil des wenigstens einen hydrophilen und/oder gewebeadhäsiven Polymers in den Fasern von der ersten Außenfläche des Produktes in Richtung der zweiten, vorzugsweise gegenüberliegenden Außenfläche des Produktes ab, vorzugsweise graduell ab.

Der Anteil des wenigstens einen synthetischen und bioresorbierbaren Polymers und/oder des wenigstens einen hydrophilen und/oder gewebeadhäsiven Polymers in den Fasern kann sich in einer weiteren Ausführungsform zwischen einer ersten Außenflächenschicht des Produktes und einer zweiten, vorzugsweise gegenüberliegenden Außenflächenschicht des Produktes ändern, vorzugsweise graduell ändern, d.h. einem Gradienten folgen.

In einer weitergehenden Ausführungsform nimmt der Anteil des wenigstens einen synthetischen und bioresorbierbaren Polymers in den Fasern zwischen einer ersten Außenflächenschicht des Produktes und einer zweiten, vorzugsweise gegenüberliegenden Außenflächenschicht des Produktes in Richtung der zweiten Außenflächenschicht zu, vorzugsweise graduell zu, und/oder der Anteil des wenigstens einen hydrophilen und/oder gewebeadhäsiven Polymers in den Fasern zwischen einer ersten Außenflächenschicht des Produktes und einer zweiten, vorzugsweise gegenüberliegenden Außenflächenschicht des Produktes in Richtung der zweiten Außenflächenschicht ab, vorzugsweise graduell ab. Die erste Außenflächenschicht wird vorzugsweise von rotationsgesponnenen Fasern gebildet, welche wenigstens ein hydrophiles und/oder gewebeadhäsives Polymer im Sinne der vorliegenden Erfindung, jedoch kein synthetisches und bioresorbierbares Polymer im Sinne der vorliegenden Erfindung enthalten. Die zweite Außenflächenschicht wird dagegen bevorzugt von rotationsgesponnenen Fasern gebildet, welche wenigstens ein synthetisches und bioresorbierbares Polymer im Sinne der vorliegenden Erfindung, jedoch kein hydrophiles und/oder gewebeadhäsives Polymer im Sinne der vorliegenden Erfindung enthalten.

Besonders bevorzugt nimmt der Anteil des wenigstens einen synthetischen und bioresorbierbaren Polymers in den Fasern zwischen einer ersten Außenflächenschicht des Produktes und einer zweiten, vorzugsweise gegenüberliegenden Außenflächenschicht des Produktes in Richtung der zweiten Außenflächenschicht zu, vorzugsweise graduell zu, und entsprechend der Anteil des wenigstens einen hydrophilen und/oder gewebeadhäsiven Polymers in den Fasern zwischen der ersten Außenflächenschicht des Produktes und der zweiten, vorzugsweise gegenüberliegenden Außenflächenschicht des Produktes in Richtung der zweiten Außenflächenschicht ab, vorzugsweise graduell ab. Die erste Außenflächenschicht wird vorzugsweise von rotationsgesponnenen Fasern gebildet, welche wenigstens ein hydrophiles und/oder gewebeadhäsives Polymer im Sinne der vorliegenden Erfindung, jedoch kein synthetisches und bioresorbierbares Polymer im Sinne der vorliegenden Erfindung enthalten. Die zweite Außenflächenschicht wird dagegen bevorzugt von rotationsgesponnenen Fasern gebildet, welche wenigstens ein synthetisches und bioresorbierbares Polymer im Sinne der vorliegenden Erfindung, jedoch kein hydrophiles und/oder gewebeadhäsives Polymer im Sinne der vorliegenden Erfindung enthalten.

In einer weiteren Ausführungsform umfasst das medizinische Produkt eine Abfolge von rotationsgesponnenen Faserschichten, wobei sich der Faseranteil des wenigstens einen synthetischen und bioresorbierbaren Polymers und/oder des wenigstens einen hydrophilen und/oder gewebeadhäsiven Polymers entlang der Schichtabfolge, d.h. entlang der Abfolge der rotationsgesponnenen Faserschichten, ändert, vorzugsweise graduell ändert.

Bevorzugt nimmt der Faseranteil des wenigstens einen synthetischen und bioresorbierbaren Polymers entlang der Schichtabfolge zu, vorzugsweise graduell zu, und/oder der Faseranteil des wenigstens einen hydrophilen und/oder gewebeadhäsiven Polymers entlang der Schichtabfolge ab, vorzugsweise graduell ab.

Besonders bevorzugt nimmt der Faseranteil des wenigstens einen synthetischen und bioresorbierbaren Polymers entlang der Schichtabfolge zu, vorzugsweise graduell zu, und entsprechend der Faseranteil des wenigstens einen hydrophilen und/oder gewebeadhäsiven Polymers entlang der Schichtabfolge ab, vorzugsweise graduell ab.

In einer bevorzugten Ausführungsform umfasst das medizinische Produkt zwischen einer ersten Außenflächenschicht und einer zweiten, vorzugsweise gegenüberliegenden Außenflächenschicht eine Abfolge von rotationsgesponnenen Faserschichten, bei welchen der Faseranteil des wenigstens einen synthetischen und bioresorbierbaren Polymers entlang der Schichtabfolge in Richtung der zweiten Außenflächenschicht zunimmt, vorzugsweise graduell zunimmt, und/oder der Faseranteil des wenigstens einen hydrophilen und/oder gewebeadhäsiven Polymers entlang der Schichtabfolge in Richtung der zweiten Außenflächenschicht abnimmt, vorzugsweise graduell abnimmt. Besonders bevorzugt nimmt der Faseranteil des wenigstens einen synthetischen und bioresorbierbaren Polymers entlang der Schichtabfolge in Richtung der zweiten Außenflächenschicht zu, vorzugsweise graduell zu, und entsprechend der Faseranteil des wenigstens einen hydrophilen und/oder gewebeadhäsiven Polymers entlang der Schichtabfolge in Richtung der zweiten Außenflächenschicht ab, vorzugsweise graduell ab. Die erste Außenflächenschicht wird vorzugsweise von rotationsgesponnenen Fasern gebildet, welche wenigstens ein hydrophiles und/oder gewebeadhäsives Polymer im Sinne der vorliegenden Erfindung, jedoch kein synthetisches und bioresorbierbares Polymer im Sinne der vorliegenden Erfindung enthalten. Die zweite Außenflächenschicht wird dagegen bevorzugt von rotationsgesponnenen Fasern gebildet, welche wenigstens ein synthetisches und bioresorbierbares Polymer im Sinne der vorliegenden Erfindung, jedoch kein hydrophiles und/oder gewebeadhäsives Polymer im Sinne der vorliegenden Erfindung enthalten.

Bei dem in den vorangegangenen Abschnitten erwähnten Gradienten kann es sich um einen kontinuierlichen oder diskontinuierlichen, insbesondere stufenförmigen, Gradienten handeln.

Die in den vorangegangenen Ausführungsformen erwähnten/erwähnte Schichten/wenigstens eine Schicht können/kann eine Dicke von 10 µm bis 4000 µm, insbesondere 100 µm bis 4000 µm, bevorzugt 500 µm bis 2000 µm, aufweisen.

Die Fasern können einen Anteil an dem wenigstens einen synthetischen und bioresorbierbaren Polymer von 1 Gew.-% bis 99 Gew.-%, insbesondere 20 Gew.-% bis 99 Gew.-%, bevorzugt 50 Gew.-%bis 99 Gew.-%, aufweisen, bezogen auf das Gesamtgewicht einer Einzelfaser.

Des Weiteren können die Fasern einen Anteil an dem wenigstens einen hydrophilen und/oder gewebeadhäsiven Polymer von 1 Gew.-% bis 99 Gew.-%, insbesondere 1 Gew.-% bis 80 Gew.-%, bevorzugt 1 Gew.-% bis 50 Gew.-%, aufweisen, bezogen auf das Gesamtgewicht einer Einzelfaser.

Das medizinische Produkt weist in einer weiteren Ausführungsform einen Faseranteilsgradient in Bezug auf das wenigstens eine synthetische und bioresorbierbare Polymer von 100 Gew.-% bis 30 Gew.-%, insbesondere 100 Gew.-% bis 50 Gew.-%, bevorzugt 100 Gew.-% bis 70 Gew.-%, auf pro Einzelfaser. Das medizinische Produkt kann insbesondere einen Faseranteilsgradient in Bezug auf das wenigstens eine synthetische und bioresorbierbare Polymer von 90 Gew.-% bis 30 Gew.-%, bevorzugt 80 Gew.-% bis 60 Gew.-%, pro Einzelfaser aufweisen.

In einer weiteren Ausführungsform weist das medizinische Produkt einen Faseranteilsgradient in Bezug auf das wenigstens eine hydrophile und/oder gewebeadhäsive Polymer von 100 Gew.-% bis 30 Gew.-%, insbesondere 100 Gew.-% bis 50 Gew.-%, bevorzugt 100 Gew.-% bis 70 Gew.-%, auf pro Einzelfaser. Das medizinische Produkt kann insbesondere einen Faseranteilsgradient in Bezug auf das wenigstens eine hydrophile und/oder gewebeadhäsive Polymer von 90 Gew.-% bis 30 Gew.-%, bevorzugt 80 Gew.-% bis 60 Gew.-%, pro Einzelfaser aufweisen.

Das wenigstens eine synthetische und bioresorbierbare Polymer ist ausgewählt aus der Gruppe umfassend Polylactid, Polyglycolid, Poly-ε-caprolacton, Polytrimethylencarbonat, Poly-3-hydroxybutyrat, Poly-4-hydroxybutyrat, Poly-para-dioxanon, Copolymere davon, Derivate davon, Stereoisomere davon und Mischungen (Blends) davon.

Das wenigstens eine hydrophile und/oder gewebeadhäsive Polymer ist ausgewählt aus der Gruppe umfassend Polyacrylsäure, Polyvinylpyrrolidinone, Proteine, Polysaccharide, insbesondere Cellulosen, Mucopolysaccharide, Copolymere davon, Derivate davon, Stereoisomere davon, Salze davon und Mischungen (Blends) davon.

Das wenigstens eine hydrophile und/oder gewebeadhäsive Polymer kann insbesondere ausgewählt sein aus der Gruppe umfassend Cellulose, Methylcellulose (als Lebensmittelzusatzstoff unter der Nummer E 461 zugelassen), Ethylcellulose (als Lebensmittelzusatzstoff unter der Nummer E 462 zugelassen), Hydroxypropylcellulose (als Lebensmittelzusatzstoff unter der Nummer E 463 zugelassen), Hydroxypropylmethylcellulose (als Lebensmittelzusatzstoff unter der Nummer E 464 zugelassen), Methylethylcellulose (als Lebensmittelzusatzstoff unter der Nummer E 465 zugelassen), Natriumcarboxymethylcellulose (als Lebensmittelzusatzstoff unter der Nummer E466 zugelassen), Hydroxyethylcellulose, Hydroxybutylmethylcellulose, Celluloseacetat, Celluloseacetatbutyrat, Celluloseacetatmaleat, Celluloseacetatphthalat, Celluloseacetattrimellitat, Cellulosefettsäureester (insbesondere Cellulosedilaurat, Cellulosedipalmitat, Cellulosedistearat, Cellulosemonopalmitat, Cellulosemonostearat, Cellulosetrilaurat, Cellulosetripalmitat und/oder Cellulosetristearat), Agar, Alginsäure, Ammoniumalginat, Natriumalginat, Kalziumalginat, Kalzium- und Natriumsalze von Cellulosecarboxymethylether, Carrageenan, i-Carrageenan, κ-Carrageenan, λ-Carrageenan, Stärke, acetylierte Stärke, Distärkephosphat, insbesondere acetylierte Distärkephosphat, vorgelatinisierte Stärke, Johannisbrotkernmehl, Maisstärke, Quellstärke, Pullulan, Dextrin, Cellulose-2-hydroxyethylether, Hydroxyethylmethylcellulose, Cellulose-2-hydroxypropylether, Cellulose-2-hydroxypropylether (niedriger Substitutionsgrad), Hydroxypropylstärke, Ethanolhomopolymer, Hyaluronsäure, Natriumhyaluronat, Gelatine, Copolymere davon, Derivate davon, Stereoisomere davon, Salze davon und Mischungen davon.

Gemäß einer weiteren Ausführungsform können die Fasern wenigstens ein Additiv enthalten, welches vorzugsweise ausgewählt ist aus der Gruppe umfassend Weichmacher, Füllstoffe, Vernetzungsmittel, Farbstoffe, medizinische Wirkstoffe und Mischungen davon.

Insbesondere können die Fasern einen Anteil an dem wenigsten einen Additiv von 0 Gew.-% bis 10 Gew.-%, insbesondere 0.05 Gew.-% bis 10 Gew.-%, bevorzugt 0.1 Gew.-% bis 5 Gew.-%, weiter bevorzugt 1 Gew.-% bis 5 Gew.-%, aufweisen, bezogen auf das Gesamtgewicht einer Einzelfaser.

Geeignete Weichmacher können ausgewählt sein aus der Gruppe umfassend Alkohole, Zuckeralkohole, Polyalkohole, Polyether, Natriumcelluloseglycolate, Polyvinylacetat, Polyvinylpyrrolidin und Mischungen davon.

Geeignete Füllstoffe können ausgewählt sein aus der Gruppe umfassend vernetzter und insbesondere niedermolekularer Polyvinylalkohol, vernetztes Polyvinylacetat, vernetztes Polyvinylpyrrolidon (z.B. erhältlich unter der Bezeichnung Polyblasdone™), Polyvinylharnstoffe, Dextrane, Natriumcellulose, Glykolat, Crosskaramellose (z.B. unter der Bezeichnung Ac-Die-Sol™ erhältlich), Hydroxyalkylcellulosen, wie beispielsweise Hydroxypropylmethylcellulose, Hydroalkylcellulosen, wie beispielsweise Hydroxyethylcellulose, Alkylcellulosen, wie beispielsweise Methylcellulose, Mikrocellulose (z.B. unter der Bezeichnung Avicel™ erhältlich), Pektin, Gellan, Alginate, Ionenaustauscher, beispielsweise auf Basis von Methacrylatcopolymeren mit Divinylbenzol (z.B. erhältlich unter der Bezeichnung Tulsion™), und Mischungen davon.

Als geeignete Farbstoffe kommen grundsätzlich zugelassene Farbstoffe aus dem Arzneimittel-, Kosmetik- oder Lebensmittelbereich in Frage. Beispielsweise können die Farbstoffe ausgewählt sein aus der Gruppe umfassend Curcumin, Riboflavin, Quinolingelb, Tartrazin, Gelborange S, Karmin, Azorubin, Ponceau 4R, Erythrosin, Red 2G, Allulared AC, Patent Blau V, Indigotin, Brilliantblau FCF, Chlorophyll, Karamell, Braun FK, Brilliantschwarz BN, Anthocyanine, Xanthophylle, Carotinoide, D & C Farbstoffe und Mischungen davon.

Geeignete medizinische Wirkstoffe können ausgewählt sein aus der Gruppe umfassend antimikrobielle, insbesondere antibiotische, Wirkstoffe, desinfizierende Wirkstoffe, entzündungshemmende Wirkstoffe, blutstillende Wirkstoffe, geruchsbekämpfende Wirkstoffe und Mischungen davon.

In einer bevorzugten Ausführungsform weist das medizinische Produkt einen Faseranteilsgradient in Bezug auf das wenigstens eine Additiv auf. Dadurch ist mit besonderem Vorteil eine graduelle Einstellung von zusätzlichen funktionalen Produkteigenschaften möglich, wie beispielsweise von Flexibilität, mechanischer Stabilität, Färbung und/oder medizinischer Wirksamkeit.

Der Anteil des wenigstens einen Additivs kann sich insbesondere von einer ersten Außenfläche des Produktes in Richtung einer zweiten, vorzugsweise gegenüberliegenden Außenfläche des Produktes ändern, vorzugsweise graduell ändern, d.h. einem Gradienten folgen. Mit anderen Worten ist ein Faseranteilsgradient in Bezug auf das wenigstens eine Additiv vorzugsweise entlang der Dicke des Produktes ausgebildet.

Dabei kann der Anteil des wenigstens einen Additivs in den Fasern von der ersten Außenfläche des Produktes in Richtung der zweiten, vorzugsweise gegenüberliegenden Außenfläche des Produktes zu- oder abnehmen, vorzugsweise graduell zu- oder abnehmen.

In einer weiteren Ausführungsform umfasst das medizinische Produkt eine Abfolge von rotationsgesponnenen Faserschichten, wobei sich der Faseranteil des wenigstens einen Additivs entlang der Schichtabfolge ändert, insbesondere zu- oder abnimmt.

Bevorzugt umfasst das medizinische Produkt eine Abfolge von rotationsgesponnenen Faserschichten, wobei sich der Faseranteil des wenigstens einen Additivs entlang der Schichtabfolge graduell ändert, insbesondere graduell zu- oder abnimmt.

Bei dem in den vorangegangenen Abschnitten erwähnten Gradienten kann es sich ebenfalls um einen kontinuierlichen oder diskontinuierlichen, insbesondere stufenförmigen, Gradienten handeln.

Zur Erhöhung der mechanischen Stabilität des medizinischen Produktes können die Fasern mechanisch verfestigt sein. Eine solche Verfestigung kann beispielsweise auf statistischen Verschlingungen oder Verdrillungen der Fasern, die obendrein die Dehnbarkeit des Produktes in feuchtem Zustand verbessern, beruhen. Alternativ oder in Ergänzung hierzu können die Fasern mittels Wasserstrahlvernadeln, Pressen und/oder Kalandrieren mechanisch verfestigt sein.

Bevorzugt besitzt das medizinische Produkt eine Reißfestigkeit (Höchstzugspannung) von wenigstens 0.15 N/mm². Das Produkt kann insbesondere eine Reißfestigkeit von 0.15 N/mm² bis 3 N/mm², bevorzugt 0.5 N/mm² bis 2 N/mm², weiter bevorzugt von 1 N/mm², aufweisen.

Die Fasern weisen in einer weiteren Ausführungsform einen Durchmesser von 50 nm bis 200 µm, insbesondere 100 nm bis 150 µm, bevorzugt 200 nm bis 100 µm, auf (bestimmt mittels Rasterelektronenmikroskopie als Mittelwert von 50 Einzelfasern).

In einer weiteren Ausführungsform unterscheiden sich die Fasern gegenüberliegender Außenflächenschichten des Produktes in ihrem Durchmesser.

Der Durchmesser der Fasern kann sich insbesondere von einer ersten Außenfläche des Produktes in Richtung einer zweiten, vorzugsweise gegenüberliegenden Außenfläche des Produktes ändern, vorzugsweise graduell ändern, d.h. einem Gradienten folgen. Mit anderen Worten ist ein Faserdurchmessergradient vorzugsweise entlang der Dicke des Produktes ausgebildet.

Dabei kann der Faserdurchmesser von der ersten Außenfläche des Produktes in Richtung der zweiten, vorzugsweise gegenüberliegenden Außenfläche des Produktes zu- oder abnehmen, vorzugsweise graduell zu- oder abnehmen.

In einer weiteren Ausführungsform umfasst das medizinische Produkt eine Abfolge von rotationsgesponnenen Faserschichten, wobei sich der Durchmesser der Fasern entlang der Schichtabfolge ändert, insbesondere zu- oder abnimmt.
Bevorzugt umfasst das medizinische Produkt eine Abfolge von rotationsgesponnenen Faserschichten, wobei sich der Durchmesser der Fasern entlang der Schichtabfolge graduell ändert, insbesondere graduell zu- oder abnimmt.

Bei dem in den vorangegangenen Abschnitten erwähnten Gradienten kann es sich ebenfalls um einen kontinuierlichen oder diskontinuierlichen, insbesondere stufenförmigen, Gradienten handeln.

Im feuchten Zustand kann das medizinische Produkt eine Dehnbarkeit von 50% bis 400%, insbesondere 100% bis 300%, bevorzugt 150% bis 200%, aufweisen, bezogen auf die ursprüngliche Länge des Produktes (im trockenen und nicht gedehnten Zustand).

Das medizinische Produkt kann weiterhin eine Dicke von 0.1 mm bis 10 mm, insbesondere 0.2 mm bis 6 mm, bevorzugt 0.5 mm bis 3 mm, aufweisen (bestimmt nach ISO 9073-2).

Das medizinische Produkt kann ferner ein Flächengewicht von 10 g/m² bis 300 g/m², insbesondere 20 g/m² bis 250 g/m², bevorzugt 40 g/m² bis 250 g/m², aufweisen.

Das medizinische Produkt ist in einer weiteren Ausführungsform im trockenen Zustand offenporig ausgebildet.

Insbesondere kann das medizinische Produkt im trockenen Zustand eine Luftdurchlässigkeit von 0.1 l/[min cm²] bis 1 l/[min cm²], insbesondere 0.3 l/[min cm²] bis 0.8 l/[min cm²], bevorzugt 0.3 l/[min cm²] bis 0,7 l/[min cm²], aufweisen (bestimmt nach EN ISO 9237).

Das medizinische Produkt ist bevorzugt innerhalb eines Zeitraumes von zwei Jahren, vorzugsweise einem Jahr, insbesondere sechs Monaten, in vivo resorbierbar (bioresorbierbar).

Das medizinische Produkt ist weiterhin vorzugsweise schichtförmig ausgebildet oder besitzt vorzugsweise einen schichtförmigen Aufbau.

Das medizinische Produkt kann grundsätzlich eine textile Struktur besitzen, insbesondere ein textiles Flächengebilde aufweisen oder in Form eines solchen Gebildes vorliegen.

Zum Beispiel kann das medizinische Produkt ein Gestrick, Gewirk, Gewebe oder Geflecht aufweisen oder in Form einer solchen Textilstruktur vorliegen.

Weiterhin kann das medizinische Produkt einen pseudomonofilen oder multifilen, insbesondere geflochtenen oder gezwirnten, Faden aufweisen oder in Form eines solchen Fadens vorliegen.

Erfindungsgemäß bevorzugt ist es indes, wenn das medizinische Produkt eine nicht textile Struktur, insbesondere ein nicht textiles Flächengebilde aufweist oder in Form eines solchen Gebildes vorliegt.

Das medizinische Produkt liegt in Form eines Vlieses vor.

Besonders bevorzugt liegt das medizinische Produkt in Form eines Vliesstoffes vor.

Weiterhin bevorzugt ist es, wenn es sich bei dem medizinischen Produkt um ein Implantat handelt oder das Produkt zur Verwendung als Implantat vorgesehen ist.

Das medizinische Produkt ist besonders bevorzugt zur Versiegelung oder Verklebung von biologischem, vorzugsweise menschlichem oder tierischem, Gewebe und/oder von Wunden, insbesondere inneren Wunden, vorgesehen. Das Produkt kann insbesondere zur Approximation von Gewebehälften, die beispielsweise in Folge einer Operation voneinander getrennt worden sind, eingesetzt werden.

Das medizinische Produkt eignet sich weiterhin vorzugsweise zur Verwendung als Hämostyptikum, d.h. als Blutstillungsmittel. Es ist insbesondere zur Stillung von starken Blutungen, insbesondere von Blutungen von parenchymalen Organen, wie beispielsweise Lunge, Niere, Milz und/oder Leber, geeignet.

Weiterhin kann das medizinische Produkt als Absorptionsmittel für Körperflüssigkeiten, insbesondere Blut, Exsudat, Eiter, Liquor und/oder Lymphe, vorgesehen sein.

Des Weiteren kann das medizinische Produkt zur Anwendung bei der Versiegelung von Flüssigkeits- und/oder Gasleckagen im Körper eines menschlichen oder tierischen Patienten vorgesehen sein. Bei den Flüssigkeitsleckagen kann es sich insbesondere um Blut-, Exsudat-, Eiter-, Liquor- und/oder Lymphleckagen handeln. Bei den Gasleckagen handelt es sich bevorzugt um Luftleckagen, insbesondere der Lunge.

Eine weitere bevorzugte Anwendung betrifft die Verwendung des medizinischen Produktes als Anti-Adhäsionsmittel, d.h. als Mittel zur Verhinderung und/oder Prophylaxe von postoperativen Gewebeadhäsionen/-verklebungen.

Ferner kann das medizinische Produkt als Keimbarriere, insbesondere zur Verhinderung von post-chirurgischen Infektionen, verwendet werden.

Weitere Anwendungen betreffen die Verwendbarkeit des medizinischen Produktes als Wundbrücke zur Vermeidung von Wunddehiszenzen, zur Abdeckung eines Duradefektes, als Faszienersatz, als Schwellkörper zur Gefäßkompression und/oder Gewebeüberlappung, als Matrix für die in vitro und/oder in vivo Zellbesiedelung und/oder als Platzhalter für körpereigenes Gewebe.

In einem zweiten Aspekt betrifft die Erfindung ein Verfahren zur Herstellung eines medizinischen Produktes, wie es im Rahmen des ersten Erfindungsaspekts beschrieben worden ist.

Das erfindungsgemäße Verfahren zeichnet sich dadurch aus, dass ausgehend von einem Faserrohmaterial, welches wenigstens ein erstes synthetisches und bioresorbierbares Polymer und wenigstens ein zweites hydrophiles und/oder gewebeadhäsives Polymer enthält, mittels Rotationsspinnen Fasern erzeugt werden.

In einer weiteren Ausführungsform werden ferner ausgehend von einem Faserrohmaterial, welches wenigstens ein synthetisches und bioresorbierbares Polymer im Sinne der vorliegenden Erfindung, jedoch kein hydrophiles und/oder gewebeadhäsives Polymer im Sinne der vorliegenden Erfindung enthält, mittels Rotationsspinnen Fasern erzeugt.

In einer weiteren Ausführungsform werden ferner ausgehend von einem Faserrohmaterial, welches wenigstens ein hydrophiles und/oder gewebeadhäsives Polymer im Sinne der vorliegenden Erfindung, jedoch kein synthetisches und bioresorbierbares Polymer im Sinne der vorliegenden Erfindung enthält, mittels Rotationsspinnen Fasern erzeugt.

Das Faserrohmaterial liegt zweckmäßigerweise in fluider oder fluidisierter Form vor. Grundsätzlich kann das Faserrohmaterial in Form einer Schmelze, Lösung, Suspension oder Dispersion bereitgestellt werden.

Das Faserrohmaterial kann einen Anteil an dem wenigstens einen synthetischen und bioresorbierbaren Polymer von 1 Gew.-% bis 99 Gew.-%, insbesondere 20 Gew.-% bis 99 Gew.-%, bevorzugt 50 Gew.-% bis 99 Gew.-%, aufweisen, bezogen auf das Gesamtgewicht des Faserrohmaterials.

Das Faserrohmaterial kann weiterhin einen Anteil an dem wenigstens einen hydrophilen und/oder gewebeadhäsiven Polymer von 1 Gew.-% bis 99 Gew.-%, insbesondere 1 Gew.-% bis 80 Gew.-%, bevorzugt 1 Gew.-% bis 50 Gew.-%, enthalten, bezogen auf das Gesamtgewicht des Faserrohmaterials.

In einer bevorzugten Ausführungsform wird das Faserrohmaterial zur Erzeugung der Fasern in einen Vorlagebehälter gegeben, wobei der Behälter in Rotation versetzt wird und wobei das Faserrohmaterial durch Zentripetalkräfte aus dem Behälter in Form von Fasern ausgetragen wird.

Die austretenden Fasern können gerichtet, kontaktlos geführt werden. Eine kontaktlose und definierte Führung der Fasern, bevor diese auf eine Ablageeinrichtung auftreffen, erlaubt mit besonderem Vorteil eine Modifikation der Fasern. So kann allein die Dauer der Führung bzw. die Richtung der Führung Einfluss auf die Faserlänge, den Faserdurchmesser sowie die Faserstruktur nehmen. Eine gerichtete kontaktlose Führung erzeugt ein homogeneres Faserspektrum als ein Herstellungsprozess ohne Führung. Mit besonderem Vorteil kann allein durch die Führung die Breite einer Verteilungskurve sämtlicher Fasereigenschaften eingestellt werden. Hierdurch kann die Menge an Fasern mit unerwünschter Fasergeometrie erheblich reduziert werden.

Die Fasern werden in einer weiteren Ausführungsform über vorzugsweise als Durchgänge im Bodenbereich des oben erwähnten Vorlagebehälters ausgebildete Austrittsbereiche aus diesem ausgetragen. Die Austrittsbereiche können einen Durchmesser von 0.1 mm bis 2 mm, bevorzugt 0.3 mm bis 1 mm, insbesondere 0.3 mm bis 0.7 mm, aufweisen.

Die Austrittsbereiche des Vorlagebehälters können beispielsweise einen gegenseitigen Abstand von 10 mm aufweisen.

In einer zweckmäßigen Ausführungsform wird der Vorlagebehälter auf eine Temperatur von 20 °C bis 180 °C, insbesondere 20 °C bis 100 °C, bevorzugt 30 °C bis 60 °C, weiter bevorzugt auf 45°C, erhitzt.

Der Vorlagebehälter kann mit einer Geschwindigkeit von 1 U/min bis 25000 U/min, insbesondere 2000 U/min bis 10000 U/min, bevorzugt 3000 U/min bis 7000 U/min, rotiert werden.

Zur Ausbildung eines Faserdurchmessergradients ist es in einer weiteren Ausführungsform vorgesehen, dass die Rotationsgeschwindigkeit des Vorlagebehälters graduell verändert wird.

Die Fasern werden in einer zweckmäßigen Ausführungsform auf einer Ablageeinrichtung gesammelt.

In einer weiteren Ausführungsform kann die Fasererzeugung durch Anlegen eines elektrischen Feldes, beispielsweise zwischen einer Ablageeinrichtung und einem in Rotation versetzbaren Vorlagebehälter, insbesondere wie in den vorangegangenen Ausführungsformen beschrieben, unterstützt werden.

In einer vorteilhaften Ausführungsform können die Fasern durch eine Absaugeinrichtung geführt werden. Hierdurch sind eine zusätzliche Verstreckung oder Verdrillung der Fasern und damit eine Erhöhung der Einzelfaserfestigkeit erzielbar. Erfindungsgemäß ist es denkbar, dass die Fasern durch einen Gasstrom transportiert werden. Als Gas kann beispielsweise Luft verwendet werden. Alternativ oder in Ergänzung können Inertgase, wie beispielsweise Stickstoff, verwendet werden.

Der Anteil des wenigstens einen synthetischen und bioresorbierbaren Polymers und/oder des wenigstens einen hydrophilen und/oder gewebeadhäsiven Polymers in dem Faserrohmaterial wird während des Rotationsspinnens graduell, insbesondere kontinuierlich oder diskontinuierlich, beispielsweise stufenweise, verändert.

Dies kann beispielsweise dadurch realisiert werden, dass ein in Rotation versetzbarer Vorlagebehälter mit unterschiedlichen Förderraten mit einer ersten, das wenigstens eine synthetische und bioresorbierbare Polymer enthaltenden Flüssigkeit und einer zweiten, das wenigstens eine hydrophile und/oder gewebeadhäsive Polymer enthaltenden Flüssigkeit beschickt wird. Dabei kann die Förderrate der ersten Flüssigkeit graduell erhöht und die Förderrate der zweiten Flüssigkeit entsprechend graduell erniedrigt werden oder umgekehrt. Alternativ kann die Förderrate einer der beiden Flüssigkeiten konstant gehalten und diejenige der anderen Flüssigkeit graduell erhöht oder erniedrigt werden.

Erfindungsgemäß kann es weiterhin vorgesehen sein, dass zunächst nur eine der beiden Flüssigkeiten in einen in Rotation versetzbaren Vorlagebehälter gefördert wird, wohingegen die Förderung der anderen Flüssigkeit in den Vorlagebehälter erst in einem zeitlich definierten Abstand erfolgt. Dabei kann die Förderrate der einen Flüssigkeit zunehmend gedrosselt und insbesondere auf Null gefahren werden, wohingegen die Förderrate der anderen Flüssigkeit vorzugsweise entsprechend erhöht wird. Hierdurch lassen sich mit besonderem Vorteil medizinische Produkte mit einer ersten Außenflächenschicht und einer zweiten, vorzugsweise gegenüberliegenden Außenflächenschicht herstellen, wobei die Fasern der ersten Außenflächenschicht das wenigstens eine synthetische und bioresorbierbare Polymer, nicht jedoch das wenigstens eine hydrophile und/oder gewebeadhäsive Polymer und die Fasern der zweiten Außenflächenschicht das wenigstens eine hydrophile und/oder gewebeadhäsive Polymer, nicht jedoch das wenigstens eine synthetische und bioresorbierbare Polymer enthalten.

Bei den in den beiden vorangegangenen Abschnitten beschriebenen Flüssigkeiten kann es sich grundsätzlich um Schmelzen, Lösungen, Dispersionen und/oder Suspensionen handeln.

Das Faserrohmaterial kann ferner wenigstens ein Additiv, insbesondere mit einem Anteil von 0 Gew.-% bis 10 Gew.-%, insbesondere 0.05 Gew.-% bis 10 Gew.-%, bevorzugt 0.1 Gew.-% bis 5 Gew.-%, weiter bevorzugt 1 bis 5 Gew.-%, aufweisen, bezogen auf das Gesamtgewicht des Faserrohmaterials.

Zur Ausbildung eines Fasergradienten in Bezug auf das wenigstens eine Additiv kann der Anteil des wenigstens einen Additivs in dem Faserrohmaterial während des Rotationsspinnens graduell, insbesondere kontinuierlich oder diskontinuierlich, beispielsweise stufenweise, verändert werden.

Dies kann beispielsweise dadurch geschehen, dass eine erste, das wenigstens eine synthetische und bioresorbierbare Polymer enthaltenden Flüssigkeit und/oder eine zweite, das wenigstens eine hydrophile und/oder gewebeadhäsive Polymer enthaltende Flüssigkeit mit dem wenigstens einen Additiv versetzt werden und mit unterschiedlichen Förderraten in einen in Rotation versetzbaren Vorlagebehälter gefördert werden. Dabei kann die Förderrate der ersten Flüssigkeit graduell erhöht und die Förderrate der zweiten Flüssigkeit entsprechend graduell erniedrigt werden oder umgekehrt. Alternativ kann die Förderrate einer der beiden Flüssigkeiten konstant gehalten und diejenige der anderen Flüssigkeit graduell erhöht oder erniedrigt werden. Auch die in diesem Abschnitt erwähnten Flüssigkeiten können grundsätzlich in Form von Schmelzen, Lösungen, Dispersionen und/oder Suspensionen vorliegen.

In einer besonders vorteilhaften Ausführungsform werden die rotationsgesponnenen Fasern, vorzugsweise unter Ausbildung eines Vliesstoffes, mechanisch kompaktiert oder verfestigt. Dies kann beispielsweise mittels Wasserstrahlvernadeln und/oder Pressen erfolgen. Das Pressen der Fasern kann insbesondere mittels einer Sandstanze oder eines Kalanders erfolgen.

Bezüglich weiterer Merkmale und Vorteile des Verfahrens wird zur Vermeidung von Wiederholungen vollumfänglich auf die im Rahmen der Beschreibung des ersten Erfindungsaspekts gemachten Ausführungen Bezug genommen.

Weitere Merkmale und Vorteile der Erfindung ergeben sich aus der nachfolgenden Beschreibung bevorzugter Ausführungsformen in Form von Beispielen sowie aus den Unteransprüchen. Die bevorzugten Ausführungsformen dienen dabei lediglich dem besseren Verständnis der Erfindung, ohne diese hierauf zu beschränken.

### Beispielteil

### Beispiel 1: Herstellung eines Vlieses aus Polycaprolacton und acetyliertem Distärkephosphat

Es wurde eine erste Lösung ausgehend von 20 g Polycaprolacton (PCL) und 80 g Anisol bei 80°C unter Ultraschallexposition sowie gelegentlichem Rühren hergestellt. Eine zweite Lösung wurde ausgehend von 3 g acetylierter Distärkephosphat (6345 Spezialstärke, Fa. Südstärke) und 5 g Dimethylsulfoxid (DMSO) unter Rühren bei 80°C hergestellt.

Die beiden Lösungen wurden jeweils auf Raumtemperatur (ca. 25 °C) abgekühlt und über zwei Spritzenpumpen (Harvard Spritzenpumpen 11) einem Spinnrotor zugeführt, wo sie mit Hilfe eines Statikmischers homogenisiert wurden. Für das Rotationsspinnen wurde die Temperatur des Spinnrotors auf 35 °C eingestellt. Die Rotationsgeschwindigkeit des Spinnrotors betrug 3000 U/min. Der Boden des Spinnrotors verfügte über zwölf Austrittsöffnungen mit einem Durchmesser von 350 µm und einem gegenseitigen Abstand von 52 mm. Die Fasern wurden nach Verlassen der Austrittsöffnungen bei Raumtemperatur mit Hilfe einer Absaugungseinrichtung (750 m³/min) auf einer Ablage unterhalb des Rotors gesammelt. Zur Ausbildung eines Faseranteilsgradient in Bezug auf das Polycaprolacton und die Stärke wurde die erste Lösung mit einer konstanten Pumpgeschwindigkeit von 10 ml/min in den Spinnrotor gefördert, während die Pumpgeschwindigkeit der Stärkelösung während eines Zeitraumes von 10 min von 0 auf 7.5 ml/min graduell erhöht wurde.

Das erhaltene Vlies besaß eine Dicke von 90 µm (ISO 9073-2), ein Flächengewicht von 75 g/m² (ISO 9073-1) sowie einen Faseranteilsgradient in Bezug auf das Polycaprolacton von 100 Gew.-% bis 70 Gew.-% pro Einzelfaser. Das Vlies zeigte im trockenen Zustand eine hohe Reißfestigkeit und war im nassen Zustand stabil sowie gut positionierbar.

### Beispiel 2: Herstellung eines Vlieses aus Polycaprolacton und acetyliertem Distärkephosphat

Ein weiteres Vlies wurde entsprechend der Vorschrift von Beispiel 1 hergestellt, wobei der zweiten Lösung zusätzlich 0,5 g Polyvinylalkohol (Mowiol® 20-98) zur Stabilisierung der entstehenden Dispersion zugesetzt wurden.

Es wurde ein im trockenen Zustand reißfestes Vlies erhalten, welches in nassem Zustand ebenfalls stabil sowie positionierbar war.

### Beispiel 3: Herstellung eines Vlieses aus Polycaprolacton und kaltwasserlöslicher Stärke

Es wurde ein weiteres Vlies entsprechend der Vorschrift von Beispiel 1 hergestellt, wobei anstelle der 6345 Spezialstärke die sogenannte Zulkowsky-Stärke (Fa. Sigma-Aldrich) verwendet wurde.

Es wurde ebenfalls ein im trockenen Zustand reißfestes Vlies erhalten, welches in nassem Zustand stabil sowie positionierbar war.

### Beispiel 4: Herstellung eines Vlieses aus Polycaprolacton und kaltwasserlöslicher Stärke

Es wurde ein weiteres Vlies entsprechend der Vorschrift von Beispiel 1 hergestellt, wobei Zulkowsky-Stärke (Fa. Sigma-Aldrich) und Wasser anstelle der 6345 Spezialstärke und von DMSO verwendet wurde.

Es wurde ebenso ein im trockenen Zustand reißfestes Vlies erhalten, welches in nassem Zustand stabil sowie positionierbar war.

### Nicht erfindungsgemäßes Beispiel 5: Herstellung eines Vlieses aus Polylactid-co-glycolid und kaltwasserlöslicher Stärke

Es wurde eine erste Lösung ausgehend von 25 g Zulkowsky-Stärke (Fa. Sigma-Aldrich) und 27.5 g DMSO (48%) bei einer Temperatur von 60 °C unter Ultraschallexposition hergestellt. Eine zweite Lösung wurde ausgehend von 13 g Polylactid-Glycolid-Copolymer (Resomer RG 504H, Fa. Böhringer Ingelheim) ebenfalls bei einer Temperatur von 60 °C und unter Ultraschallexposition hergestellt.

Die beiden Lösungen wurden bei einer Lösungstemperatur von jeweils 60 °C über zwei Spritzenpumpen (Harvard Spritzenpumpen 11) einem Spinnrotor zugeführt. Für das Rotationsspinnen wurde die Temperatur des Spinnrotors auf 59 °C eingestellt. Die Rotationsgeschwindigkeit wurde graduell von 1500 auf 4000 U/min über einen Zeitraum von 15 min erhöht. Der Boden des Spinnrotors verfügte über zwölf Austrittsöffnungen mit einem Durchmesser von 350 µm und einem gegenseitigen Abstand von 52 mm. Die Fasern wurden nach Verlassen der Austrittsöffnungen bei Raumtemperatur mit Hilfe einer Absaugungseinrichtung (750 m³/min) auf einer Ablage unterhalb des Rotors gesammelt.

Das erhaltene Vlies besaß eine Dicke von 30 µm (ISO 9073-2) sowie ein Flächengewicht von 20 g/m² (ISO 9073-1). Die Fasern des abgelegten Vlieses wiesen auf der unteren Außenfläche einen mittleren Durchmesser von 1 µm und auf der gegenüberliegenden oberen Außenfläche einen mittleren Durchmesser von 200 nm auf. Das Vlies war reißfest und in nassem Zustand stabil und gut positionierbar.

### Nicht erfindungsgemäßes Beispiel 6: Herstellung eines Vlieses aus Stärke und Polycaprolacton

Ausgehend von 6 g Stärke (Spezialstärke 6345) und 90 g Anisol wurde unter Rühren eine Dispersion hergestellt. Zu der Dispersion wurden unter Rühren bei einer Temperatur von 80 °C während zwei Stunden 24 g Polycaprolacton (80 kDa, Fa. Sigma-Aldrich) hinzugegeben. Anschließend ließ man die erhaltene Lösung auf Raumtemperatur abkühlen.

Die Lösung wurde über eine Spritzenpumpe mit einer Geschwindigkeit von 2 ml/min in einen Spinnrotor (wie in der DE 10 2005 048 939 A1 beschrieben) gefördert, dessen Temperatur auf 45 °C eingestellt war. Die Rotationsgeschwindigkeit betrug 3000 U/min. Der Boden des Spinnrotors verfügte über 24 Austrittsöffnungen mit einem Durchmesser von 800 µm und einem gegenseitigen Abstand von 26 mm.

Das erhaltene Vlies besaß eine Dicke von 30 µm (ISO 9073-2), einen mittleren Faserdurchmesser von 400 nm sowie ein Flächengewicht von 20 g/m² (ISO 9073-1). Das Vlies war schwach hydrophil und erreichte seine maximale Wasseraufnahmekapazität nach wenigen Minuten. Ferner war es in nassem Zustand stabil und gut positionierbar.

### Nicht erfindungsgemäßes Beispiel 7: Herstellung eines Vlieses aus Stärke und Polycaprolacton

Es wurden 81 g Anisol und 9 g DMSO gemischt. Zu der Mischung wurden 6 g Stärke unter Rühren und unter Erhalt einer Dispersion hinzugegeben. Zu der Dispersion wurden anschließend unter Rühren bei einer Temperatur von 80 °C während zwei Stunden 24 g Polycaprolacton (80 kDa, Fa. Sigma-Aldrich) hinzugegeben. Die erhaltene Lösung ließ man auf Raumtemperatur abkühlen.

Das angewandte Rotationsspinnverfahren entsprach dem im Beispiel 6 beschriebenen Verfahren.

Das erhaltene Vlies besaß eine Dicke von 25 µm (ISO 9073-2), einen mittleren Faserdurchmesser von 400 nm sowie ein Flächengewicht von 20 g/m² (ISO 9073-1). Das Vlies war schwach hydrophil und erreichte seine maximale Wasseraufnahmekapazität innerhalb weniger Minuten. In nassem Zustand war das Vlies stabil und gut positionierbar.

### Nicht erfindungsgemäßes Beispiel 8: Herstellung eines Vlieses aus Stärke/Pullulan/Polycaprolacton

Ausgehend von 19 g Stärke (Kartoffelstärke nach Zulkowsky, Fa. Sigma-Aldrich), 1 g Pullulan (Fa. Hayashibara Co. Ltd. USP-NF) und 30 g destilliertem Wasser wurde unter Rühren bei 60 °C während einer Stunde eine erste Lösung hergestellt. Eine zweite Lösung wurde ausgehend von 10 g Polycaprolacton und 40 g Anisol unter Rühren bei einer Temperatur von 80 °C während zwei Stunden hergestellt.

Die beiden Lösungen wurden gemischt und anschließend über eine Spritzenpumpe mit einer Geschwindigkeit von 4 ml/min in einen Spinnrotor gefördert, dessen Temperatur auf 45 °C eingestellt war. Die Rotationsgeschwindigkeit betrug 3000 U/min.

Das erhaltene Vlies besaß eine Dicke von 25 µm (ISO 9073-2) und ein Flächengewicht von 30 g/m² (ISO 9073-1). Der mittlere Faserdurchmesser betrug 500 nm. Das Vlies war schwach hydrophil und erreichte seine maximale Wasseraufnahmekapazität bereits nach wenigen Minuten. Das Vlies war außerdem im feuchten Zustand stabil.

### Beispiel 9: Herstellung eines Gradientenvlieses mit einer Außenflächenschicht aus lediglich Polycaprolacton und einer gegenüberliegenden Außenflächenschicht aus Polycaprolacton und Stärke:

Die verwendeten Materialien entsprachen den im Beispiel 6 genannten Ausgangsmaterialien. Während die Herstellung der ersten Lösung gemäß Beispiel 6 erfolgte, wurden zur Herstellung der zweiten Lösung 25 g Polycaprolacton in 100 g Anisol unter Rühren bei einer Temperatur von 80 °C gelöst. Anschließend ließ man die beiden Lösungen auf Raumtemperatur abkühlen. Über zwei Spritzenpumpen wurden die beiden Lösungen in einen Spinnrotor, dessen Temperatur auf 45 °C eingestellt war, gefördert. Die Rotationsgeschwindigkeit betrug 3000 U/min. Zur Ausbildung eines Materialgradienten wurde die erste Lösung mit einer Geschwindigkeit von 0 bis 2 ml/min während drei Stunden und die zweite Lösung mit einer Geschwindigkeit von 2 bis 0 ml/min während drei Stunden in den Spinnrotor gefördert.

Das erhaltene Vlies besaß eine Dicke von 15 µm sowie ein Flächengewicht von 20 g/m². Der mittlere Faserdurchmesser betrug 400 nm. Die Fasern des Vlieses besaßen einen Materialgradienten. Das Vlies besaß zwei funktional unterscheidbare Außenflächenschichten. Während die Außenflächenschicht aus Polycaprolacton hydrophobe Eigenschaften aufwies, besaß die gegenüberliegende Außenflächenschicht aufgrund des Stärkeanteils hydrophile Eigenschaften, was sich u.a. in einer maximalen Wasseraufnahmekapazität innerhalb von nur einer Minute bemerkbar machte.

### Beispiel 10: Herstellung eines Gradientenvlieses aus Polycaprolacton/Stärke/Pullulan:

Es wurden die gleichen Materialien wie im Beispiel 8 verwendet.

Zur Herstellung einer ersten Lösung wurden 19 g Stärke und 1 g Pullulan in 30 g destilliertem Wasser unter Rühren bei einer Temperatur von 60 °C während einer Stunde gelöst. Zur Herstellung einer zweiten Lösung wurden 20 g Polycaprolacton in 80 g Anisol unter Rühren bei einer Temperatur von 80 °C während zwei Stunden gelöst.

Das Spinnrotationsverfahren wurde in diesem Fall zweistufig ausgeführt. Zunächst wurden lediglich 50 g der zweiten Lösung gemäß den im Beispiel 1 genannten Verfahrensbedingungen ausgesponnen. Anschließend wurden die erste Lösung und die zweite Lösung über Spritzenpumpen in den Spinnrotor, dessen Temperatur auf 45 °C eingestellt war, gefördert. Die Rotationsgeschwindigkeit betrug 3000 U/min. Während die erste Lösung mit einer Geschwindigkeit von 1:0.2 bis 1.8 ml/min in den Rotor gefördert wurde betrug die Fördergeschwindigkeit für die zweite Lösung 2:0.2 ml/min.

Das erhaltene Vlies besaß eine Dicke von 30 µm, einen mittleren Faserdurchmesser von 500 nm sowie ein Flächengewicht von 40 g/m². Die Fasern des Vlieses besaßen einen Materialgradienten. Das Vlies besaß zwei funktional unterscheidbare Außenflächenschichten. Während die Außenflächenschicht aus Polycaprolacton hydrophobe Eigenschaften besaß, besaß die gegenüberliegende Außenflächenschicht aufgrund des hohen Stärkeanteils hydrophile Eigenschaften, was sich u.a. in einer maximalen Wasseraufnahmekapazität innerhalb von nur einer Minute manifestierte.

### Beispiel 11: Herstellung eines Vlieses mit einer Außenflächenschicht aus Polycaprolacton und einer aus Polycaprolacton, Carboxymethylcellulose und Polyethylenglykol bestehenden Außenflächenschicht.

Bei der verwendeten Carboxymethylcellulose handelte es sich um 7H4XF Blanose der Firma Herkules (Cas.-nr.:9004-32-4). Der Substitutionsgrad (Verfahren MA 304.1506 A) variierte von 0.65 bis 0.9, der Natriumanteil lag zwischen 7.0% und 8.9% und der Schwefelascheanteil zwischen 21.6% und 27.9%.

Zur Herstellung einer ersten Lösung wurde 1 g Polyethylenoxid (Molekulargewicht: 1.000.000 g/mol) in Wasser gelöst. Zu der Lösung wurden 7.5 g 7H4XF Blanose hinzugegeben. Anschließend ließ man die Mischung während 24 Stunden bei Raumtemperatur unter Erhalt einer Lösung rühren.

Zur Herstellung einer zweiten Lösung wurden 25 g Polycaprolacton in 100 g Anisol unter Rühren bei einer Temperatur von 80 °C gelöst. Die Lösung wurde anschließend auf Raumtemperatur abgekühlt.

Danach wurden die beiden Lösungen über zwei Spritzenpumpen einem Spinnrotor zugeführt, dessen Temperatur auf 45° Celsius eingestellt war. Die Rotationsgeschwindigkeit betrug 3000 U/min. Während die erste Lösung mit einer Geschwindigkeit von 1:0 bis 2 ml/min während drei Stunden in den Spinnrotor gefördert wurde, wurde die zweite Lösung mit einer Geschwindigkeit von 2:2 bis 0 ml/min während ebenfalls drei Stunden in den Spinnrotor gefördert.

Das erhaltene Vlies besaß eine Dicke von 20 µm, einen mittleren Faserdurchmesser von 200 nm sowie ein Flächengewicht von 20 g/m². Die Fasern des Vlieses wiesen einen Materialgradienten auf. Das Vlies besaß zwei funktional unterscheidbare Außenflächenschichten. Während die Außenflächenschicht aus Polycaprolacton hydrophobe Eigenschaften besaß, verfügte die gegenüberliegende Außenflächenschicht aufgrund des Carboxymethylcelluloseanteils über hydrophile Eigenschaften. Dies zeigte sich u.a. in einer maximalen Wasseraufnahmekapazität innerhalb von nur einer Minute und einer guten Anhaftung an verschiedenen Geweben, wie beispielsweise Haut, Leber, Knochen und dergleichen.

### Beispiel 12: Herstellung eines Gradientenvlieses mit einer Außenflächenschicht aus Polycaprolacton und einer Außenflächenschicht aus Polycaprolacton/Povidone:

Zur Herstellung einer ersten Lösung wurden 40 g Polyvinylpyrrolidon (Kollidon F90, BASF AG, Deutschland) in ein Becherglas gefüllt. Danach wurden 160 g Wasser hinzugegeben. Anschließend wurde die Mischung bei Raumtemperatur während 24 Stunden gerührt und im Anschluss daran auf 80 °C während einer Stunde erhitzt. Schließlich wurde die Lösung während einer Stunde im Ultraschallbad behandelt, bevor sie auf 60 °C abgekühlt wurde.

Zur Herstellung einer zweiten Lösung wurden 25 g Polycaprolacton in 100 g Anisol unter Rühren bei 80 °C gelöst. Anschließend ließ man die Lösung auf Raumtemperatur abkühlen.

Die beiden Lösungen wurden über zwei Spritzenpumpen einem Spinnrotor zugeführt, dessen Temperatur auf 45° Celsius eingestellt war. Die Rotationsgeschwindigkeit betrug 3000 U/min. Während die erste Lösung mit einer Geschwindigkeit von 1:0 bis 2 ml/min während drei Stunden in den Spinnrotor gefördert wurde, wurde die zweite Lösung mit einer Geschwindigkeit von 2:2 bis 0 ml/min ebenfalls während drei Stunden in den Spinnrotor gefördert.

Das erhaltene Vlies besaß eine Dicke von 50 µm, einen mittleren Faserdurchmesser von 600 nm sowie ein Flächengewicht von 40 g/m². Die Fasern des Vlieses wiesen einen Materialgradienten auf. Das Vlies besaß zwei funktional unterscheidbare Außenflächenschichten. Während die Außenflächenschicht aus Polycaprolacton hydrophobe Eigenschaften besaß, verfügte die gegenüberliegende Außenflächenschicht aufgrund des Polyvinylpyrrolidonanteils über hydrophile Eigenschaften. Dies zeigte sich in einer maximalen Wasseraufnahmekapazität innerhalb von nur einer Minute und einer guten Anhaftung an verschiedenen Geweben, wie beispielsweise Haut oder Mucosa.

### Beispiel 13: Herstellung eines Vlieses mit einer Außenflächenschicht aus Polycaprolacton und einer gegenüberliegenden Außenflächenschicht aus Polycaprolacton/Polyvinylalkohol:

Zur Herstellung einer ersten Lösung wurden Polyvinylalkohol (Mowiol 20-98; Molekulargewicht: 125.000 g Promol) in destilliertem Wasser bei einer Temperatur von 60 °C unter Rühren während sechs Stunden gelöst. Anschließend wurde die Lösung auf Raumtemperatur abgekühlt.

Zur Herstellung einer zweiten Lösung wurden 25 g Polycaprolacton in 100 g Anisol unter Rühren bei 80 °C gelöst. Anschließend wurde die Lösung ebenfalls auf Raumtemperatur abgekühlt.

Die beiden Lösungen wurden über zwei Spritzenpumpen einem Spinnrotor zugeführt, dessen Temperatur auf 45 °C eingestellt war. Die Rotationsgeschwindigkeit betrug 3000 U/min. Während die erste Lösung mit einer Geschwindigkeit von 1:0 bis 2 ml/min während drei Stunden in den Spinnrotor gefördert wurde, wurde die zweite Lösung mit einer Geschwindigkeit von 2:2 bis 0 ml/min ebenfalls während drei Stunden in den Spinnrotor gefördert.

Die Fasern des erhaltenen Vlieses wiesen einen Materialgradienten auf. Das Vlies besaß zwei funktional unterscheidbare Außenflächenschichten. Während die Außenflächenschicht aus Polycaprolacton hydrophobe Eigenschaften besaß, verfügte die gegenüberliegende Außenflächenschicht aufgrund des hohen Polyvinylalkoholanteils über hydrophile Eigenschaften. Dies machte sich u.a. in einer maximalen Wasseraufnahmekapazität innerhalb von nur einer Minute bemerkbar.

### Beispiel 14: Herstellung eines Gradientenvlieses mit einer Außenflächenschicht aus Polycaprolacton und einer gegenüberliegenden Außenflächenschicht aus Polycaprolacton/Hyaluronsäure:

Zur Herstellung einer ersten Lösung wurden 12 g einer Mischung aus 66 Gew.-% Hyaluronsäure (Renovhyal; Molekulargewicht zwischen 20 kDa und 50 kDa; Firma Soliance) und 34 Gew.-% Hyaluronsäure (Cristalhyal; Molekulargewicht zwischen 1.000.000 g/mol und 1.400.000 g/mol; Firma Soliance) in 88 g Wasser bei Raumtemperatur unter Rühren während 24 Stunden gelöst.

Zur Herstellung einer zweiten Lösung wurden 25 g Polycaprolacton in 100 g Anisol unter Rühren bei einer Temperatur von 80 °C gelöst. Anschließend wurde die Lösung auf Raumtemperatur abgekühlt.

Die beiden Lösungen wurden sodann über zwei Spritzenpumpen einem Spinnrotor zugeführt, dessen Temperatur auf 45 °C eingestellt war. Die Rotationsgeschwindigkeit betrug 3000 U/min. Während die erste Lösung mit einer Geschwindigkeit von 1:0 bis 2 ml/min während drei Stunden in den Spinnrotor gefördert wurde, betrug die Fördergeschwindigkeit für die zweite Lösung während drei Stunden 2:2 bis 0 ml/min.

Das erhaltene Vlies besaß eine Dicke von 15 µm, einen mittleren Faserdurchmesser von 250 nm sowie ein Flächengewicht von 10 g/m². Die Fasern des Vlieses wiesen einen Materialgradienten auf. Das Vlies besaß zwei funktional unterscheidbare Außenflächenschichten. Während die Außenflächenschicht aus Polycaprolacton hydrophobe Eigenschaften aufwies, verfügte die gegenüberliegende Außenflächenschicht aufgrund des hohen Hyaluronsäureanteils über hydrophile Eigenschaften. Dies zeigte sich u.a. in einer maximalen Wasseraufnahmekapazität innerhalb von nur einer Minute und einer guten Anhaftung an verschiedenen Geweben, wie beispielsweise Haut, Knorpel, Knochen und dergleichen.

## Patentansprüche

1. Medizinisches Produkt in Form einer vliesförmigen Wundauflage, umfassend rotationsgesponnene Fasern, welche wenigstens ein synthetisches und bioresorbierbares Polymer und wenigstens ein hydrophiles und/oder gewebeadhäsives Polymer enthalten, **dadurch gekennzeichnet, dass** das Produkt rotationsgesponnene Faserschichten umfasst, die sich in Bezug auf den Faseranteil des wenigstens einen synthetischen und bioresorbierbaren Polymers und/oder des wenigstens einen hydrophilen und/oder gewebeadhäsiven Polymers voneinander unterscheiden, das wenigstens eine synthetische und bioresorbierbare Polymer ausgewählt ist aus der Gruppe umfassend Polylactid, Polyglycolid, Poly-e-caprolacton, Polytrimethylencarbonat, Poly-3-hydroxybutyrat, Poly-4-hydroxy-butyrat, Poly-para-dioxanon, Copolymere davon, Derivate davon, Stereoisomere davon und Mischungen davon und das wenigstens eine hydrophile und/oder gewebeadhäsive Polymer ausgewählt ist aus der Gruppe umfassend Polyacrylsäure, Polyvinylpyrrolidinone, Proteine, insbesondere Gelatine, Polysaccharide, insbesondere Cellulosen, Mucopolysaccharide, Copolymere davon, Derivate davon, Stereoisomere davon und Mischungen davon.

2. Medizinisches Produkt nach Anspruch 1, **dadurch gekennzeichnet, dass** das Produkt rotationsgesponnene Fasern umfasst, die einen im Verhältnis zueinander unterschiedlichen Anteil an dem wenigstens einen synthetischen und bioresorbierbaren Polymer und/oder dem wenigstens einen hydrophilen und/oder gewebeadhäsiven Polymer enthalten.

3. Medizinisches Produkt nach Anspruch 1 oder 2, **dadurch gekennzeichnet, dass** das Produkt wenigstens eine rotationsgesponnene Faserschicht umfasst, deren Fasern einen höheren Anteil an dem wenigstens einen synthetischen und bioresorbierbaren Polymer enthalten als an dem wenigstens einen hydrophilen und/oder gewebeadhäsiven Polymer.

4. Medizinisches Produkt nach einem der vorhergehenden Ansprüche, **dadurch gekennzeichnet, dass** das Produkt wenigstens eine rotationsgesponnene Faserschicht umfasst, deren Fasern einen kleineren Anteil an dem wenigstens einen synthetischen und bioresorbierbaren Polymer enthalten als an dem wenigstens einen hydrophilen und/oder gewebeadhäsiven Polymer.

5. Medizinisches Produkt nach einem der vorhergehenden Ansprüche, **dadurch gekennzeichnet, dass** das Produkt wenigstens eine rotationsgesponnene Faserschicht umfasst, deren Fasern das wenigstens eine synthetische und bioresorbierbares Polymer, jedoch nicht das wenigstens eine hydrophile und/oder gewebeadhäsive Polymer enthalten.

6. Medizinisches Produkt nach einem der vorhergehenden Ansprüche, **dadurch gekennzeichnet, dass** das Produkt wenigstens eine rotationsgesponnene Faserschicht umfasst, deren Fasern das wenigstens eine hydrophile und/oder gewebeadhäsive Polymer, jedoch nicht das wenigstens eine synthetische und bioresorbierbares Polymer enthalten.

7. Medizinisches Produkt nach einem der vorhergehenden Ansprüche, **dadurch gekennzeichnet, dass** das Produkt einen Faseranteilsgradient in Bezug auf das wenigstens eine synthetische und bioresorbierbare Polymer und in Bezug auf das wenigstens eine hydrophile und/oder gewebeadhäsive Polymer besitzt.

8. Medizinisches Produkt nach einem der vorhergehenden Ansprüche, **dadurch gekennzeichnet, dass** sich der Anteil des wenigstens einen synthetischen und bioresorbierbaren Polymers und/oder des wenigstens einen hydrophilen und/oder gewebeadhäsiven Polymers in den Fasern von einer ersten Außenfläche des Produktes in Richtung einer zweiten vorzugsweise gegenüberliegenden Außenfläche des Produktes graduell ändert.

9. Medizinisches Produkt nach einem der vorhergehenden Ansprüche, **dadurch gekennzeichnet, dass** sich der Anteil des wenigstens einen synthetischen und bioresorbierbaren Polymers und/oder des wenigstens einen hydrophilen und/oder gewebeadhäsiven Polymers in den Fasern zwischen einer ersten Außenflächenschicht und einer zweiten vorzugsweise gegenüberliegenden Außenflächenschicht des Produktes graduell ändert.

10. Medizinisches Produkt nach einem der vorhergehenden Ansprüche, **dadurch gekennzeichnet, dass** das Produkt eine Abfolge von rotationsgesponnenen Faserschichten umfasst, wobei sich der Faseranteil des wenigstens einen synthetischen und bioresorbierbaren Polymers und/oder des wenigstens einen hydrophilen und/oder gewebeadhäsiven Polymers entlang der Schichtabfolge graduell ändert.

11. Medizinisches Produkt nach einem der vorhergehenden Ansprüche, **dadurch gekennzeichnet, dass** die Fasern einen Anteil an dem wenigstens einen synthetischen und bioresorbierbaren Polymer von 1 Gew.-% bis 99 Gew.-%, insbesondere 20 Gew.-% bis 99 Gew.-%, bevorzugt 50 Gew.-% bis 99 Gew.-%, aufweisen, bezogen auf das Gesamtgewicht einer Einzelfaser, und die Fasern einen Anteil an dem wenigstens einen hydrophilen und/oder gewebeadhäsiven Polymer von 1 Gew.-% bis 99 Gew.-%, insbesondere 1 Gew.-% bis 80 Gew.-%, bevorzugt 1 Gew.-% bis 50 Gew.-%, aufweisen, bezogen auf das Gesamtgewicht einer Einzelfaser.

12. Medizinisches Produkt nach einem der vorhergehenden Ansprüche, **dadurch gekennzeichnet, dass** die Fasern mechanisch verfestigt sind, insbesondere mittels Wasserstrahlvernadeln, Pressen und/oder Kalandrieren.

13. Verfahren zur Herstellung eines medizinischen Produktes nach einem der vorhergehenden Ansprüche, bei welchem ausgehend von einem Faserrohmaterial, welches wenigstens ein synthetisches und bioresorbierbares Polymer, ausgewählt aus der Gruppe umfassend Polylactid, Polyglycolid, Poly-ε-caprolacton, Polytrimethylencarbonat, Poly-3-hy-droxybutyrat, Poly-4-hydroxybutyrat, Poly-para-dioxanon, Copolymere davon, Derivate davon, Stereoisomere davon und Mischungen davon, und wenigstens ein hydrophiles und/oder gewebeadhäsives Polymer, ausgewählt aus der Gruppe umfassend Polyacrylsäure, Polyvinylpyrrolidone, Proteine, insbesondere Gelatine, Polysaccharide, insbesondere Cellulosen, Mukopolysaccharide, Copolymere davon, Derivate davon, Stereoisomere davon und Mischungen davon, enthält, mittels Rotationsspinnen Fasern erzeugt werden, **dadurch gekennzeichnet, dass** der Anteil des wenigstens einen synthetischen und bioresorbierbaren Polymers und/oder des wenigstens einen hydrophilen und/oder gewebeadhäsiven Polymers in dem Faserrohmaterial während des Rotationsspinnes graduell verändert wird.

## Claims

1. Medical product in the form of a nonwoven wound dressing, comprising rotospun fibers including at least one synthetic and bioabsorbable polymer and at least one hydrophilic and/or tissue-adhesive polymer,
**characterized in that**
the product comprises rotospun fibrous layers differing from each other in relation to the fiber fraction of the at least one synthetic and bioabsorbable polymer and/or the at least one hydrophilic and/or tissue-adhesive polymer, the at least one synthetic and bioabsorbable polymer is selected from the group comprising polylactide, polyglycolide, poly-ε-caprolactone, polytrimethylene carbonate, poly-3-hydroxybutyrate, poly-4-hydroxybutyrate, poly-para-dioxanone, copolymers thereof, derivatives thereof, stereoisomers thereof and mixtures thereof, and the at least one hydrophilic and/or tissue-adhesive polymer is selected from the group comprising polyacrylic acid, polyvinylpyrrolidones, proteins, in particular gelatin, polysaccharides, in particular celluloses, mucopolysaccharides, copolymers thereof, derivatives thereof, stereoisomers thereof and mixtures thereof.

2. Medical product according to claim 1, **characterized in that** the product comprises rotospun fibers, including mutually different fractions of the at least one synthetic and bioabsorbable polymer and/or the at least one hydrophilic and/or tissue-adhesive polymer.

3. Medical product according to claim 1 or 2, **characterized in that** the product comprises at least one rotospun fibrous layer whose fibers include a higher fraction of the at least one synthetic and bioabsorbable polymer than of the at least one hydrophilic and/or tissue-adhesive polymer.

4. Medical product according to any of the preceding claims, **characterized in that** the product comprises at least one rotospun fibrous layer whose fibers include a smaller fraction of the at least one synthetic and bioabsorbable polymer than of the at least one hydrophilic and/or tissue-adhesive polymer.

5. Medical product according to any of the preceding claims, **characterized in that** the product comprises at least one rotospun fibrous layer whose fibers include the at least one synthetic and bioabsorbable polymer, but not the at least one hydrophilic and/or tissue-adhesive polymer.

6. Medical product according to any of the preceding claims, **characterized in that** the product comprises at least one rotospun fibrous layer whose fibers include the at least one hydrophilic and/or tissue-adhesive polymer, but not the at least one synthetic and bioabsorbable polymer.

7. Medical product according to any of the preceding claims, **characterized in that** the product has a fiber fraction gradient in relation to the at least one synthetic and bioabsorbable polymer and in relation to the at least one hydrophilic and/or tissue-adhesive polymer.

8. Medical product according to any of the preceding claims, **characterized in that** the fraction of the at least one synthetic and bioabsorbable polymer and/or of the at least one hydrophilic and/or tissue-adhesive polymer in the fibers gradually changes from a first outer surface of the product in the direction of a second preferably opposite outer surface of the product.

9. Medical product according to any of the preceding claims, **characterized in that** the fraction of the at least one synthetic and bioabsorbable polymer and/or of the at least one hydrophilic and/or tissue-adhesive polymer in the fibers gradually changes between a first outer surface layer and a second preferably opposite outer surface layer of the product.

10. Medical product according to any of the preceding claims, **characterized in that** the product comprises a sequence of rotospun fibrous layers, wherein the fiber fraction of the at least one synthetic and bioabsorbable polymer and/or of the at least one hydrophilic and/or tissue-adhesive polymer gradually changes along the sequence of layers.

11. Medical product according to any of the preceding claims, **characterized in that** the fibers comprise the at least one synthetic and bioabsorbable polymer in a fraction extending from 1 wt% to 99 wt%, in particular from 20 wt% to 99 wt%, preferably from 50 wt% to 99 wt%, based on the total weight of an individual fiber, and the fibers comprise the at least one hydrophilic and/or tissue-adhesive polymer in a fraction extending from 1 wt% to 99 wt%, in particular from 1 wt% to 80 wt%, preferably from 1 wt% to 50 wt%, based on the total weight of an individual fiber.

12. Medical product according to any of the preceding claims, **characterized in that** the fibers are mechanically consolidated, in particular by hydroentangling, pressing and/or calendering.

13. Method of producing a medical product according to any of the preceding claims, wherein starting from a fiber raw material including at least one synthetic and bioabsorbable polymer, selected from the group comprising polylactide, polyglycolide, poly-ε-caprolactone, polytrimethylene carbonate, poly-3-hydroxybutyrate, poly-4-hydroxybutyrate, poly-para-dioxanone, copolymers thereof, derivatives thereof, stereoisomers thereof and mixtures thereof, and at least one hydrophilic and/or tissue-adhesive polymer, selected from the group comprising polyacrylic acid, polyvinylpyrrolidones, proteins, in particular gelatin, polysaccharides, in particular celluloses, mucopolysaccharides, copolymers thereof, derivatives thereof, stereoisomers thereof and mixtures thereof, fibers are produced using rotospinning, **characterized in that** the fraction of the at least one synthetic and bioabsorbable polymer and/or the at least one hydrophilic and/or tissue-adhesive polymer in the fiber raw material is gradually varied during the rotospinning procedure.

## Revendications

1. Produit médical sous la forme d'un pansement non-tissé, comprenant des fibres filées par rotation, qui contiennent au moins un polymère synthétique et biorésorbable et au moins un polymère hydrophile et/ou adhérant aux tissus, **caractérisé en ce que** le produit comprend des couches de fibres filées par rotation qui diffèrent les unes des autres au regard de la proportion de fibres dudit au moins un polymère synthétique et biorésorbable et/ou dudit au moins un polymère hydrophile et/ou adhérant aux tissus, ledit au moins un polymère synthétique et biorésorbable est choisi dans le groupe comprenant le polylactide, le polyglycolide, la poly-ε-caprolactone, le polycarbonate de triméthylène, le poly-3-hydroxybutyrate, le poly-4-hydroxybutyrate, la poly-para-dioxanone, leurs copolymères, leurs dérivés, leurs stéréoisomères et leurs mélanges, et ledit au moins un polymère hydrophile et/ou adhérant aux tissus est choisi dans le groupe comprenant l'acide polyacrylique, la polyvinylpyrrolidone, les protéines, notamment la gélatine, les polysaccharides, notamment les celluloses, les mucopolysaccharides, leurs copolymères, leurs dérivés, leurs stéréoisomères et leurs mélanges.

2. Produit médical selon la revendication 1, **caractérisé en ce que** le produit comprend des fibres filées par rotation, qui contiennent une proportion différente les unes par rapport aux autres dudit au moins un polymère synthétique et biorésorbable et/ou dudit au moins un polymère hydrophile et/ou adhérant aux tissus.

3. Produit médical selon la revendication 1 ou 2, **caractérisé en ce que** le produit comprend au moins une couche de fibres filées par rotation dont les fibres contiennent une proportion plus élevée dudit au moins un polymère synthétique et biorésorbable que dudit au moins un polymère hydrophile et/ou adhérant aux tissus.

4. Produit médical selon l'une quelconque des revendications précédentes, **caractérisé en ce que** le produit comprend au moins une couche de fibres filées par rotation dont les fibres contiennent une proportion plus faible dudit au moins un polymère synthétique et biorésorbable que dudit au moins un polymère hydrophile et/ou adhérant aux tissus.

5. Produit médical selon l'une quelconque des revendications précédentes, **caractérisé en ce que** le produit comprend au moins une couche de fibres filées par rotation dont les fibres contiennent ledit au moins un polymère synthétique et biorésorbable, mais toutefois pas ledit au moins un polymère hydrophile et/ou adhérant aux tissus.

6. Produit médical selon l'une quelconque des revendications précédentes, **caractérisé en ce que** le produit comprend au moins une couche de fibres filées par rotation dont les fibres contiennent ledit au moins un polymère hydrophile et/ou adhérant aux tissus, mais toutefois pas ledit au moins un polymère synthétique et biorésorbable.

7. Produit médical selon l'une quelconque des revendications précédentes, **caractérisé en ce que** le produit présente un gradient de la proportion de fibres au regard dudit au moins un polymère synthétique et biorésorbable et au regard dudit au moins un polymère hydrophile et/ou adhérant aux tissus.

8. Produit médical selon l'une quelconque des revendications précédentes, **caractérisé en ce que** la proportion dudit au moins un polymère synthétique et biorésorbable et/ou dudit au moins un polymère hydrophile et/ou adhérant aux tissus dans les fibres change graduellement depuis une première surface extérieure du produit dans la direction d'une deuxième surface extérieure du produit, de préférence opposée.

9. Produit médical selon l'une quelconque des revendications précédentes, **caractérisé en ce que** la proportion dudit au moins un polymère synthétique et biorésorbable et/ou dudit au moins un polymère hydrophile et/ou adhérant aux tissus dans les fibres change graduellement entre une première couche de surface extérieure et une deuxième couche de surface extérieure du produit, de préférence opposée.

10. Produit médical selon l'une quelconque des revendications précédentes, **caractérisé en ce que** le produit comprend une succession de couches de fibres filées par rotation, la proportion de fibres dudit au moins un polymère synthétique et biorésorbable et/ou dudit au moins un polymère hydrophile et/ou adhérant aux tissus changeant graduellement le long de la succession de couches.

11. Produit médical selon l'une quelconque des revendications précédentes, **caractérisé en ce que** les fibres présentent une proportion dudit au moins un polymère synthétique et biorésorbable de 1 % en poids à 99 % en poids, notamment de 20 % en poids à 99 % en poids, de préférence de 50 % en poids à 99 % en poids, par rapport au poids total d'une fibre individuelle, et les fibres présentent une proportion dudit au moins un polymère hydrophile et/ou adhérant aux tissus de 1 % en poids à 99 % en poids, notamment de 1 % en poids à 80 % en poids, de préférence de 1 % en poids à 50 % en poids, par rapport au poids total d'une fibre individuelle.

12. Produit médical selon l'une quelconque des revendications précédentes, **caractérisé en ce que** les fibres sont consolidées mécaniquement, notamment par aiguilletage par jets d'eau, compression et/ou calandrage.

13. Procédé de fabrication d'un produit médical selon l'une quelconque des revendications précédentes, selon lequel, à partir d'une matière première fibreuse, qui contient au moins un polymère synthétique et biorésorbable, choisi dans le groupe comprenant le polylactide, le polyglycolide, la poly-ε-caprolactone, le polycarbonate de triméthylène, le poly-3-hydroxybutyrate, le poly-4-hydroxybutyrate, la poly-para-dioxanone, leurs copolymères, leurs dérivés, leurs stéréoisomères et leurs mélanges, et au moins un polymère hydrophile et/ou adhérant aux tissus, choisi dans le groupe comprenant l'acide polyacrylique, la polyvinylpyrrolidone, les protéines, notamment la gélatine, les polysaccharides, notamment les celluloses, les mucopolysaccharides, leurs copolymères, leurs dérivés, leurs stéréoisomères et leurs mélanges, des fibres sont formées par filage par rotation, **caractérisé en ce que** la proportion dudit au moins un polymère synthétique et biorésorbable et/ou dudit au moins un polymère hydrophile et/ou adhérant aux tissus dans la matière première fibreuse est changée graduellement pendant le filage par rotation.
